# EUROPÄISCHE PATENTSCHRIFT

(11) **EP 3 985 633 B1**
(45) Veröffentlichungstag und Bekanntmachung des Hinweises auf die Patenterteilung: **29.11.2023**
(21) Anmeldenummer: 21201931.9
(22) Anmeldetag: 11.10.2021
(51) Int. Cl.: G08B 21/14, G01N 33/00, G08B 21/16, G08B 29/14

(54) **SENSOREINHEIT, GASWARNGERÄT, GASWARNVORRICHTUNG, VERFAHREN ZUM DURCHFÜHREN EINER WARTUNGSHANDLUNG AN DEM GASWARNGERÄT UND VERFAHREN ZUM DURCHFÜHREN EINER GASMESSUNG MIT DEM GASWARNGERÄT**
SENSOR UNIT, GAS WARNING UNIT, GAS WARNING DEVICE, METHOD FOR CARRYING OUT MAINTENANCE ON THE GAS WARNING DEVICE AND METHOD FOR CARRYING OUT A GAS MEASUREMENT WITH THE GAS WARNING DEVICE
UNITÉ DE CAPTEUR, APPAREIL D'AVERTISSEMENT DE FUITE DE GAZ, DISPOSITIF D'AVERTISSEMENT DE FUITE DE GAZ, PROCÉDÉ DE MISE EN UVRE D'UNE OPÉRATION D'ENTRETIEN SUR L'APPAREIL D'AVERTISSEMENT DE FUITE DE GAZ ET PROCÉDÉ DE MISE EN UVRE D'UNE MESURE DE GAZ POURVU À L'AIDE DU DISPOSITIF D'AVERTISSEMENT DE FUITE DE GAZ

(30) Priorität: 13.10.2020 DE 102020006295
(43) Veröffentlichungstag der Anmeldung: 20.04.2022
(73) Patentinhaber: Kundo xT GmbH, 78112 St. Georgen (DE)
(72) Erfinder: Fehrenbacher, Klaus-Dieter, 78730 Lauterbach (DE); Kieninger, Thomas, 78112 St. Georgen (DE)
(74) Vertreter: mepat Patentanwälte

(56) Entgegenhaltungen:
- DE-U1- 29 723 859
- TW-U- M 589 766
- US-A1- 2010 057 401
- US-A1- 2018 267 003

## Beschreibung

Die Erfindung betrifft eine Gaswarngerät mit einer Sensoreinheit, eine Gaswarnvorrichtung, ein Verfahren zum Durchführen einer Wartungshandlung an dem Gaswarngerät und ein Verfahren zum Durchführen einer Gasmessung mit dem Gaswarngerät.

Aus dem Stand der Technik sind Gaswarngeräte bekannt, die bei Vorhandensein eines oder mehrerer Gase bzw. bei Überschreiten eines Grenzwerts ein Warnsignal abgeben. Solche Gaswarngeräte werden eingesetzt, um Personen vor Vergiftung oder Erstickung zu schützen, oder auch zum Brand- und Explosionsschutz von Gebäuden und Anlagen, um das Personal vor einer mit dem Vorhandensein des Gases verbundenen Gefahr zu warnen. Diese Geräte können am Körper mitgetragen werden, häufiger werden sie jedoch an Raumwänden oder -decken installiert, um die Raumluft zu überwachen.

Für eine möglichst hohe Sicherheit und Zuverlässigkeit der Geräte ist es erforderlich, die darin verbauten Sensoren regelmäßig auf Funktionsfähigkeit zu überprüfen oder auszutauschen. Für den Funktionstest muss den Sensoren ein Testgas zugeführt werden, das mittels einer Tauchkappe am schnellen Entweichen aus der unmittelbaren Messumgebung des Sensors gehindert wird und somit bei korrekter Funktion ein Warnsignal auslöst. Für den Sensoraustausch müssen das Gehäuse geöffnet, die Sensoren entnommen und die ausgetauschten Sensoren mit der Elektronik des Gehäuses verdrahtet werden. Dabei werden die Sensoren neu eingelernt, ehe das Gerät wieder betriebsbereit ist. Um die Wartung bzw. den Tausch zu beschleunigen, werden daher oft die Geräte als Gesamtheit ausgetauscht.

DE 103 29 834 A1 beschreibt eine Gaswarnvorrichtung, die ein Gaswarngerät als Basiseinheit mit elektrischen Versorgungs- und Datenleitungen für die Kommunikation mit einer Zentrale umfasst. Die Basiseinheit wird über Schnellverschlüsse mit einer Messeinheit, die einen Sensor und einen Mikroprozessor zur Messsignalauswertung und -verarbeitung aufweist, verbunden.

US 2010/057401 offenbart ein Gastestsystem und Testmethoden um die Funktion von Gassensoren zu testen.

Ausgehend von diesem Stand der Technik ist es Aufgabe der vorliegenden Erfindung, ein Gaswarngerät bereit zu stellen, das einfach und schnell gewartet werden kann.

Diese Aufgabe wird durch ein Gaswarngerät mit den Merkmalen des Anspruchs 1 gelöst. Die Aufgabe, eine Gaswarnvorrichtung, deren Wartung zeit- und montageoptimiert ist, bereit zu stellen, wird durch die Gaswarnvorrichtung mit den Merkmalen des Anspruchs 16 gelöst.

Weiter ergibt sich die Aufgabe, ein verbessertes Verfahren zum Warten von Gaswarnvorrichtungen bereit zu stellen.

Diese Aufgabe wird durch das Verfahren zum Durchführen einer Wartungshandlung mit den Merkmalen des unabhängigen Anspruchs 17 gelöst.

Ferner wird die weitere Aufgabe, eine vereinfachte und zuverlässige Gasmessung durchzuführen, durch das Verfahren zum Durchführen einer Gasmessung mit den Merkmalen des Anspruchs 18 gelöst.

Weiterbildungen und bevorzugte Ausführungsformen des Gaswarngeräts und der Gaswarnvorrichtung sowie der Verfahren zur Wartung und Gasmessung mit dem Gaswarngerät sind in den Unteransprüchen ausgeführt.

Gemäß einer ersten erfindungsgemäßen Ausführungsform des erfindungsgemäßen Gaswarngerätes, das eine lösbar darin angeordneteSensoreinheit aufweist, hat ein Gehäuse mit einem Gehäusedeckel, einem Gehäusekörper und einem Gehäuseunterteil. Dabei hat das Gehäuse in dem Gehäusedeckel eine Ausnehmung mit einem Boden, in dem eine elektronische Schnittstelle vorliegt. Die Sensoreinheit hat eine kreiszylindrische Aufnahmevorrichtung, wobei in der kreiszylindrischen Aufnahmevorrichtung der Gassensor und eine oder mehrere Steuerungs- und Datenverarbeitungseinheit(en) angeordnet sind. Jede Steuerungs- und Datenverarbeitungseinheit steht in kommunikativer Verbindung mit dem Gassensor. Erfindungsgemäß hat die Sensoreinheit eine an der Aufnahmevorrichtung vorliegende elektronische Schnittstelle, die dazu ausgebildet ist, in einer Benutzungsanordnung, in der die Sensoreinheit lösbar in der Ausnehmung des Gehäuses aufgenommen ist, mit der korrespondierenden elektronischen Schnittstelle kommunikativ verbunden zu werden.

Die Sensoreinheit weist einen Deckel auf, der eine Abdeckplatte hat, die ferner einen Testgaszugang aufweist, durch den ein Fluidpfad für das Testgas zu dem Gassensor bereitgestellt wird.

Das Gehäuse und die kreiszylindrische Aufnahmevorrichtung stellen einen anderen Fluidpfad für ein Messgas zu dem Gassensor bereit.

Vorteilhaft ist die Steuerungs- und Datenverarbeitungseinheit der Sensoreinheit dazu ausgebildet, die Gasmessung mittels des Gassensors zu initiieren, auszulesen und auszuwerten. Die komplette Datenverarbeitung kann damit innerhalb der Sensoreinheit stattfinden, so dass die Sensoreinheit bereits bearbeitete Daten bspw. an eine datenverarbeitende Zentraleinheit weitergeben kann. Es müssen keine Rohdaten versendet oder an einer anderen Stelle ausgewertet werden. Kommunikative Verbindung mit der Zentraleinheit meint daher jede Art von elektronischer Datenübertragung.

Vorteilhaft kann die Sensoreinheit des erfindungsgemäßen Gaswarngeräts einfach und schnell ausgetauscht werden, da sie durch die Entriegelung der beiden miteinander gekoppelten Verriegelungsmittel lösbar in dem Gehäuse des Gaswarngeräts angeordnet ist: Dazu weist die Ausnehmung erste Verriegelungsmittel auf und die Sensoreinheit weist zweite Verriegelungsmittel auf, die mit den ersten Verriegelungsmitteln lösbar in Eingriff gebracht werden können.

Das Gehäuse und die Aufnahmevorrichtung der Sensoreinheit stellen den Fluidpfad für das Messgas zu dem Gassensor bereit, da zwischen Gehäuse und der Aufnahmevorrichtung ein Eintrittsspalt für das Gas gebildet wird.

Die elektronische Schnittstelle des Bodens ist in einer Betriebsanordnung, in der die Sensoreinheit lösbar in der Ausnehmung des Gehäuses aufgenommen ist, mit der an der Aufnahmevorrichtung vorliegenden elektronischen Schnittstelle kommunikativ verbunden; es findet also eine Signalübertragung statt. Übertragen werden können u. a. Werte der Sensoreinheit oder auch einfache Steuerbefehle. Die Schnittstelle dient auch der Stromversorgung der Sensoreinheit. Die an der Aufnahmevorrichtung vorliegende elektronische Schnittstelle ist wiederum mit der elektronischen Schnittstelle der Steuerungs- und Datenverarbeitungseinheit kommunikativ verbunden.

Diese Anordnung der Sensoreinheit innerhalb des Gehäuses bildet also das erfindungsgemäße Gaswarngerät.

Durch die elektronischen Schnittstellen, die bevorzugt als Stecksysteme oder auch kontaktlos ausgebildet sind, lässt sich der Gassensor auch einfach elektronisch mit weiteren Komponenten verbinden, die in dem Gehäuse vorliegen können, so dass der Austausch der Sensoreinheit, aber auch der weiteren Komponenten, werkzeuglos erfolgen kann. So kann für die Steuerungs- und Datenverarbeitungseinheit der Sensoreinheit eine elektronische Verbindung zu der Zentraleinheit hergestellt werden. Die Sensoreinheit wird einfach eingesteckt und mittels der Verriegelungselemente in der Ausnehmung verrastet.

"Elektronische Schnittstelle" meint hierin jede Art von Verbindung zwischen zwei korrespondierenden daten(über)tragenden elektrischen Verbindungsleitungen. Die elektronischen Schnittstellen können jede Art von Steckverbindern sein, z. B. USB-Verbinder, oder auch andere Datenübertragungsverbinder, die der Fachmann als geeignet ansieht. So sind auch drahtlose bzw. kontaktlose Datenübertragungen möglich.

Die Einbettung des Sensors in eine Sensoreinheit mit vollständiger datenverarbeitender Elektronik gibt dem Benutzer die Möglichkeit, einen Sensor besonders schnell und einfach mit dem Gehäuse zu koppeln bzw. im Ganzen austauschen zu können. Die Sensoreinheit bildet damit eine separate Wechseleinheit.

Der Begriff "Betriebsanordnung" oder "Benutzungsanordnung" hierin meint, dass die Sensoreinheit in das Gehäuse eingesetzt und elektrisch sowie elektronisch mit weiteren elektronischen Komponenten verbunden ist, so dass eine Gasmessung oder eine Testmessung durchgeführt werden kann.

Der Begriff "Fluidpfad für ein Messgas" meint hierin einen durchgängigen Strömungsweg, durch den das Messgas ungehindert strömen kann, der durch die Geometrie der Bauteile labyrinthartig ist. Durch die labyrinthartige Führung des Fluidpfades wird vorteilhaft ein Spritzschutz für den Sensor erzielt, da Spritzwasser oder Feuchtigkeit von außen den Sensor nicht erreichen können, der geschützt innerhalb der Sensoreinheit vorliegt und nicht frei liegt. Ist die Sensoreinheit in der Ausnehmung des Gehäuses aufgenommen, liegt zwischen beiden Bauteilen ein Spalt vor.

In einer weiteren Ausführungsform weist die Sensoreinheit des erfindungsgemäßen Gaswarngeräts in der kreiszylindrischen Aufnahmevorrichtung ein Messrohr auf, das eine Messstrecke für ein Messgas in dem Fluidpfad zu dem Gassensor bereitstellt. Der Gassensor ist in dieser Ausführungsform ein optischer Gassensor, wobei ferner eine geeignete Licht- oder Strahlungsquelle zur Ausleuchtung der Messstrecke und eine elektrische Verbindung zu der Steuerungs- und Datenverarbeitungseinheit vorgesehen sind. Die Lichtquelle strahlt thermische Strahlung im Infrarot- oder Ultraviolett-Bereich ab, der geeignet ist, um bspw. CO₂ detektieren zu können. Es können unterschiedliche Sensoren in der Sensoreinheit verbaut sein, wie z. B. Halbleiter-, elektrochemische, fotoakustische Sensoren sowie weitere Sensoren.

In einer bevorzugten Ausführungsform weist die kreiszylindrische Aufnahmevorrichtung ein Ringelement mit einem Bodenabschnitt auf, in dem eine Ausnehmung vorliegt, durch die der Fluidpfad für das Messgas verläuft. Die Ausnehmung ist dazu ausgebildet, einen Durchtritt für die elektronische Schnittstelle des Bodens des Gehäusedeckels zur Verbindung mit der elektronischen Schnittstelle der Sensoreinheit zu schaffen. Ferner hat die kreiszylindrische Aufnahmevorrichtung eine kreiszylindrische Grundplatte, die außenumfänglich mehrere Haltemittel wie bspw. Haltebügel aufweist, die dazu dienen, die Sensoreinheit in der Gehäuseausnehmung zu halten bzw. zu positionieren. In der Grundplatte liegt eine Ausnehmung vor, die einen Durchtritt für die elektronische Schnittstelle des Bodens des Gehäusedeckels zur Verbindung mit der elektronischen Schnittstelle bildet. Es gibt mehrere Durchtrittsöffnungen in der Grundplatte, die dazu ausgebildet sind, das Messgas durchtreten zu lassen - sie sind Teil des Fluidpfades. Um die kreiszylindrische Aufnahmevorrichtung zu bilden, sitzt das Ringelement auf der Grundplatte auf, bevorzugt auf einem verdickten Umfangsrand (an dessen Außenseite die Haltemittel sind) der Grundplatte. Die Durchtrittsöffnungen können umfänglich an der Grundplatte verteilt sein oder auch in der Mitte liegen. Das Messgas kann so vorteilhaft gleichmäßig in die Sensoreinheit strömen.

In einer weiteren Ausführungsform des erfindungsgemäßen Gaswarngeräts weist das Ringelement an seiner dem Bodenabschnitt zugewandten Kante einen außenumfänglichen Flansch auf. "Flansch" meint hierbei allgemein einen um den Ring umlaufenden Vorsprung als Element zum Positionieren, Dichten, Verbinden oder Schließen der Aufnahmevorrichtung mit dem Deckel. Der Deckel hat einen kreiszylindrischen Mantel, der die Abdeckplatte trägt und der sich gegen den Flansch abstützt. Bevorzugt stützt sich der Mantel auf einem auf dem Flansch anliegenden Dichtmittel, besonders bevorzugt auf einem Dichtring ab. Der Mantel bildet für das Messgas von außen kommend eine Strömungshilfe und weist ferner Rastelemente auf, die mit korrespondierenden Haltemitteln, wie etwa den Haltebügeln an der Grundplatte, in Eingriff gebracht werden können.

In noch einer weiteren Ausführungsform des erfindungsgemäßen Gaswarngeräts ist ein Drehring außenumfänglich um den Mantel des Deckels der Sensoreinheit angeordnet. Er kann in der Betriebsanordnung gedreht werden. Um den Drehring vor Spritzwasser zu schützen, wird der Drehring bevorzugt durch die Abdeckplatte überkragt und liegt in der Betriebsanordnung um einen Rand der Ausnehmung in dem Gehäuse an, wobei er bevorzugt mittels eines außenumfänglichen Umgriffs des Drehrings drehbar auf dem Rand aufliegt, um eine weitere Windung des Messgas-Fluidpfades zu erzeugen. Der Drehring weist zwei oder mehr Typen Positionierelemente, bevorzugt drei Typen Positionierelemente auf, die in Zusammenwirkung mit Positionierelementen, die das Gehäuse aufweist, eine Position des Drehrings in der Ausnehmung bestimmen. Durch die Positionierelemente wird es möglich, den Drehring in unterschiedliche Stellungen zu bringen und zu halten und den Messgas-Fluidpfad für das Messgas durchgängig zu machen oder zu schließen.

Nach einer weiteren Ausführungsform des erfindungsgemäßen Gaswarngeräts sind die Positionierelemente aller Typen erste Platten, zweite Platten und dritte Platten, die sich von einer zu der Grundplatte weisenden Unterseite des Drehrings in Richtung der Grundplatte erstrecken. Dabei sind die ersten Platten radial näher zu einem Mittelpunkt des Drehringes an der Unterseite des Drehrings angeordnet als die zweiten Platten, bevorzugt an einem inneren Umfang des Drehrings. Sie sind durch Lücken voneinander beabstandet, genauso sind auch die zweiten Platten durch Lücken voneinander beabstandet. Auch diese befinden sich an der Unterseite des Drehrings, bevorzugt an dessen äußerem Umfang. Die Lücken zwischen den ersten Platten und die Lücken zwischen den zweiten Platten sind nicht kongruent.

Besonders bevorzugt sind in Bezug auf eine axiale Erstreckung der Platten die zweiten Platten kürzer als die ersten Platten, um mit weiteren Komponenten, die an der Ausnehmung vorliegen, einen weiteren Abschnitt des labyrinthartigen Fluidpfades zu bilden. Die Platten sind damit auf zwei Kreisringen angeordnet, die unterschiedliche Radien haben. Die Platten sind umfänglich auf diesen beiden Kreisringen verteilt und liegen auf diesen äquidistant zueinander vor. Die entlang den Kreisringen angeordneten Platten sind versetzt zueinander angeordnet, um in dem Zusammenspiel der Platten und der Positionierelemente des Gehäuses den Fluidpfad für das Messgas zu bilden bzw. diesen Fluidpfad durchgängig bereitzustellen.

In einer weiteren Ausführungsform des erfindungsgemäßen Gaswarngeräts erstrecken sich die dritten Platten von einer zu der Grundplatte weisenden Unterseite des Drehrings in Richtung der Grundplatte. Dabei sind die dritten Platten in Bezug auf die radiale Anordnung zu einem Mittelpunkt des Drehringes zwischen den ersten Platten und den zweiten Platten angeordnet und durch Lücken voneinander beabstandet an der Unterseite des Drehrings angeordnet. Die Lücken zwischen den ersten Platten und die Lücken zwischen den zweiten Platten sind mit den dritten Lücken bevorzugt nicht kongruent, damit der Fluidpfad je nach Einstellung des Drehrings geöffnet oder geschlossen werden kann.

In Bezug auf eine axiale Erstreckung der Platten sind die dritten Platten kürzer als die ersten Platten, damit eine Drehung des Drehrings ermöglicht wird, ohne dass sich die unterschiedlichen Komponenten von Drehring und Ausnehmung des Gehäusedeckels während der Drehung des Drehrings gegenseitig behindern. Die dritten Platten sind ebenfalls entlang einem Kreisring angeordnet und liegen dort auch zueinander äquidistant vor.

Alle drei Plattentypen sind in Bezug auf ihre radiale Anordnung versetzt zueinander angeordnet, so dass je nach Drehringposition der Fluidpfad für das Messgas durchgängig oder nicht durchgängig gemacht werden kann. Der Abstand der gedachten Kreisringe ergibt eine Art ringförmige Nut, die der Breite des Randes der Ausnehmung des Gehäuses entspricht, so dass der Rand in dieser Nut aufgenommen werden kann und ein Umgriff des Randes entsteht. Der Umgriff bildet eine Windung des Messgas-Fluidpfades, so dass das Messgas in die Ausnehmung und weiter in die Sensoreinheit geführt werden kann.

Ferner sind in noch einer weiteren Ausführungsform des erfindungsgemäßen Gaswarngeräts die Positionierelemente, die an dem Außenumfang des Randes der Ausnehmung vorliegen, durch Lücken voneinander beabstandet. Der Rand der Ausnehmung bildet einen Kreisring, auf dem die Positionierelemente bevorzugt äquidistant zueinander angeordnet sind. Die Positionierelemente bilden Rast- und Fluidleitelemente zugleich, so dass der Drehring verschiedene Positionen entlang des Umfangs des Randes der Ausnehmung einnehmen kann. Die Positionierelemente bilden dabei Wegbegrenzer, die eine Bewegung des Drehrings bevorzugt in zwei Positionen, offen bzw. geschlossen, begrenzen.

Nach noch einer weiteren Ausführungsform des erfindungsgemäßen Gaswarngeräts liegen Haltestege auf dem Boden an einer inneren Mantelfläche der Ausnehmung des Gehäuses verteilt vor. Die Haltestege verlaufen längsaxial in Bezug zu der Mantelfläche, die mit den Haltemitteln der kreiszylindrischen Grundplatte zum lösbaren Halten der Sensoreinheit in der Ausnehmung in Eingriff stehen. Die Haltemittel sind bevorzugt Haltebügel, deren Form mit der Form der Haltestege korrespondiert. Die Bügel und die Haltestege sind kongruent in ihrer Position und Ausformung und bilden zusammen Nut und Schiene, so dass die Haltebügel über die Haltestege geschoben werden können und so eine verdrehsichere Halterung der Sensoreinheit in der Ausnehmung des Gehäuses bilden.

Gemäß noch einer weiteren Ausführungsform des erfindungsgemäßen Gaswarngeräts liegt auf einem Boden des Gehäuses in dem Gehäusedeckel ein kreisförmiger Steg vor, der mit einem Abstand von der inneren Mantelfläche der Ausnehmung angeordnet ist. Außerdem hat die Grundplatte der Sensoreinheit an ihrer zu dem Boden weisenden Seite umfangsnah eine Nut, die es erlaubt, dass das Gas zu zumindest einem Teil der Durchtrittsöffnungen der Grundplatte gelangt. Die Nut kann den kreisförmigen Steg des Bodens der Ausnehmung des Gehäuses aufnehmen, wobei zwischen Nut und Steg ein Spalt verbleibt. Der Steg ist zu der Mantelfläche der Ausnehmung umfangsnah auf dem Boden angeordnet. Dadurch und durch den Spalt zwischen Nut und Steg wird für den Fluidpfad des Messgases eine weitere Teilstrecke des labyrinthartigen Fluidpfads gebildet.

Sollte dennoch Spritzwasser in die Ausnehmung vordringen, was bei sehr feuchter Umgebung der Fall sein kann, sieht eine weitere Ausführungsform vor, dass die Ausnehmung an einer geeigneten Stelle Austrittsöffnungen oder eine Abflussrinne aufweist, in der sich eindringende Flüssigkeit sammeln und aus der Ausnehmung und von den elektronischen Komponenten von Sensoreinheit bzw. Gehäuse abfließen kann. Ferner ist in einer Betriebsanordnung die Ausnehmung in Bezug zum Fußboden eines Raumes so orientiert, dass sie sich parallel dazu erstreckt. Wenn die Ausnehmung in dem Gehäuse mit einer Neigung gestaltet ist, kann dann in der Betriebsanordnung eindringendes Wasser ohne weiteres abfließen.

Gemäß einer weiteren Ausführungsform des erfindungsgemäßen Gaswarngeräts können die ersten Verriegelungsmittel in der Ausnehmung an einer inneren Mantelfläche vorliegende und radial nach innen weisende Elemente sein wie etwa Vorsprünge oder Nasen.

Die zweiten Verriegelungsmittel, die die Sensoreinheit aufweist, liegen an zwei oder mehreren Positionierelementen (zweite Platten) des Drehrings vor und können als Längsstege ausgebildet sein, die an einer Außenseite der zweiten Platten vorliegen. Diese untergreifen bei einem Eingriff die Vorsprünge oder Nasen, eine schienenartige Verbindung wird gebildet, wobei "schienenartig" meint, dass sowohl die Nasen als auch die Längsstege sich parallel zueinander erstrecken und beim Einschieben der Sensoreinheit in die Ausnehmung aneinander anliegen. Hierdurch kann die Sensoreinheit in dem Gehäuse gehalten werden und der Drehring kann bedient werden, ohne dass die Sensoreinheit sich in der Ausnehmung des Gehäuses drehen kann.

Die Verriegelungselemente bewirken ferner, dass die Sensoreinheit nur in einer bevorzugten, vorbestimmten Position in die Ausnehmung eingesetzt werden kann. Ferner wird dies durch den in der Ausnehmung geführten Vorsprung erreicht, der eine eindeutige Positionierung der kreissymmetrischen Komponenten gestattet. Die Verbindung der elektrischen Schnittstellen, d. h. das ineinander Stecken der Steckverbindung, kann dadurch einfach und sicher erfolgen, die elektrischen Schnittstellen werden durch diese Führung vor Beschädigungen geschützt.

Gemäß einer weiteren erfindungsgemäßen Ausführungsform des erfindungsgemäßen Gaswarngeräts ist der Drehring der Sensoreinheit in Bezug zu der Ausnehmung in verschiedene Positionen anordenbar: So kann der Drehring in eine Offenposition gebracht bzw. gedreht werden, in der der Fluidpfad des Messgases von außerhalb des Gaswarngerät bis hin zu dem Gassensor durchgängig ist, wobei in der Offenposition die Positionierelemente am Rand der Ausnehmung und die Positionierelemente des Drehrings miteinander fluchten, d. h. die Positionierelemente des Drehrings, hier die zweiten Platten, verdecken die Positionierelemente des Randes, wenn dem Fluidpfad für das Messgas gefolgt wird. Die entsprechenden Lücken zwischen den Positionierelementen des Drehrings liegen dann an der gleichen Stelle, wie die Positionierelemente des Randes der Ausnehmung, so dass Messgas in den Spalt zwischen Ausnehmung und Sensoreinheit eintreten kann.

Ferner kann der Drehring in eine Geschlossenposition gebracht bzw. gedreht werden, in der der Fluidpfad für das Messgas von außerhalb der Sensoreinheit bis hin zu dem Gassensor nicht durchgängig ist, wobei die Positionierelemente des Randes der Ausnehmung und die Positionierelemente des Drehrings, hier bspw. die zweiten Platten, umfänglich in Bezug zu dem Rand der Ausnehmung zueinander versetzt positioniert sind. Umgekehrt zur Offenposition liegen in dieser Drehringposition die Lücken der Positionierelemente des Drehrings nicht an der gleichen Stelle wie die Lücken der Positionierelemente des Randes der Ausnehmung. Messgas kann so nicht in den Spalt zwischen Ausnehmung und Sensoreinheit eintreten und nicht zu dem Sensor geführt werden.

Bevorzugt kann durch die spezifische Anordnung der Positionier- und Verriegelungselemente von Drehring und Gehäuseausnehmung zueinander der Drehring zwei Positionen einnehmen, die Offen- und die Geschlossenposition. Die Drehung in jede Richtung ist begrenzt durch die genannte Anordnung der Positionier- und Verriegelungselemente.

Um die Sensoreinheit einfach austauschen zu können, kann der Drehring ferner in eine Austauschposition gebracht bzw. gedreht werden, in der die ersten Verriegelungsmittel nicht mit den zweiten Verriegelungsmitteln in Eingriff stehen bzw. gelöst werden. Die Verriegelungsmittel können über Entriegelungselemente gelöst werden, wobei die Entriegelungselemente derart gestaltet sind, dass sie ergonomisch leicht erreicht und bedient werden können, damit die Sensoreinheit mit einer Hand werkzeuglos aus der Ausnehmung herausgenommen werden kann. Die Verriegelungselemente sind als Federelement ausgebildet und weisen ein Kugelelement auf, so dass sie bei Montage leicht einrasten und auch leicht entrastet werden können, wenn die Sensoreinheit ausgetauscht werden soll.

Gemäß einer Ausführungsform des erfindungsgemäßen Gaswarngeräts weist der Drehring einen oder mehrere Griffe auf, wobei ein Griff an einem äußeren Umfang des Drehrings vorliegt. Mit dem Griff kann der Drehring in die beschriebenen Positionen verstellt werden, wobei ein Benutzer dies einfach einhändig tun kann. Die Stellung des Griffs ermöglicht es ferner, einem Bediener anzuzeigen, in welcher Position sich der Drehring befindet. Dies kann auf dem Gehäuse auch angezeigt werden, bspw. durch in das Material des Gehäuses eingebrachte oder aufgeklebte Piktogramme, so dass bei diesem Anzeigebeispiel durch das Drehen des Drehrings und Einstellen des Griffs auf die Position entsprechend eines Piktogramms sofort der Betriebsmodus der Sensoreinheit klar ersichtlich ist.

In noch einer weiteren Ausführungsform des erfindungsgemäßen Gaswarngeräts ist in der Aufnahmevorrichtung eine Batterie angeordnet, die eine Uhr betreiben kann, die in der Sensoreinheit vorliegt und die die Zeitspanne misst, wie lange die Sensoreinheit in Betrieb ist.

Ferner kann das erfindungsgemäße Gaswarngerät eine Ausgabevorrichtung wie eine optische oder eine akustische Ausgabevorrichtung aufweisen, die nach einer Ausführungsform in der Sensoreinheit bzw. an dem Gehäuse vorliegen kann und mit der Steuerungs- und Datenverarbeitungseinheit der Sensoreinheit kommunikativ verbunden ist. Die Ausgabevorrichtung kann Zustände der Sensoreinheit (aktuell/Warnzustand) bzw. erfasste Messwerte des Gassensors bzw. Positionsdaten, die von Positionssensoren erfasst werden, die an dem Gehäuse vorliegen und dazu ausgebildet sind, eine jeweilige Position des Drehrings zu erfassen, und die mit der Steuerungseinheit kommunikativ verbunden sind, an einen Benutzer ausgeben, indem diese einen Ton, Töne, Geräusche oder Audiosequenzen abgibt, oder indem eine Warnleuchte leuchtet, die etwa in dem Deckel der Sensoreinheit oder anderen Teilen des Gehäuses vorliegt.

Wenn eine Leuchte vorliegt, kann bspw. ein Farbcode Auskunft geben, ob eine Warnsituation vorliegt (hohe Messwerte) oder eine Wartung der Sensoreinheit aufgrund eines turnusmäßigen Tests erfolgen muss oder sogar ein Austausch der gesamten Sensoreinheit aufgrund eines fehlerhaften Sensors.

Gemäß einer noch weiteren Ausführungsform des erfindungsgemäßen Gaswarngeräts weist das Gehäuse eine elektronische Eingabetaste, bspw. kapazitive Taste auf. Die Taste kann bspw. dazu dienen, einen durch die Ausgabevorrichtung ausgegebenen Zustand der Sensoreinheit zu quittieren, oder Eingaben damit vorzunehmen.

Gemäß einer bevorzugten Ausführungsform des erfindungsgemäßen Gaswarngeräts sind in der Sensoreinheit bzw. dem Gehäuse Positionssensoren zur Erfassung der unterschiedlichen Positionen des Drehrings angeordnet. Die Positionssensoren sind mit der Steuerungs- und Datenverarbeitungseinheit zur Weitergabe der erfassten Positionen kommunikativ verbunden. Bevorzugt handelt es sich bei den Positionssensoren um Reed-Kontakte, es können aber auch andere einfache Lage- bzw. Kontaktsensoren eingesetzt werden. Dabei schaltet die Steuerungseinheit eine Betriebskonfiguration der Sensoreinheit frei, wenn der Drehring in einer Offenposition erfasst wird. Wird eine Geschlossenposition des Drehrings festgestellt, schaltet die Steuerungseinheit eine Testkonfiguration der Sensoreinheit frei. Beim Erfassen einer Austauschposition kann das Gaswarngerät ebenfalls in eine entsprechende Austauschkonfiguration versetzt werden, so dass, wenn eine neue Sensoreinheit eingesetzt wird, diese sogleich erfasst und angesteuert wird, um sie einzulernen.

Ferner kann die Steuerung- und Datenverarbeitungseinheit auf der Sensoreinheit in einer weiteren Ausführungsform des erfindungsgemäßen Gaswarngeräts aus mehreren Teilen aufgebaut sein. So liegen in der Sensoreinheit eine Sensorplatine mit Elektronik zum Steuern und zum Auslesen des Sensors, eine Platine mit Elektronik für die optischen akustischen Ausgabevorrichtung und ggf. eine separate Platine mit Kommunikationselektronik vor, die mit einer weiteren Elektronik in dem übrigen Gehäuse kommunizieren kann. Vorteilhaft wird die gesamte Datenverarbeitung der Messdaten mit den elektronischen Komponenten innerhalb der Sensoreinheit durchgeführt. In dem Gehäusekörper ist eine weitere Platine mit Kommunikationselektronik für die Verbindung der Sensoreinheit mit einer entsprechenden Peripherie, wie z. B. der Zentraleinheit, angeordnet.

Ferner kann das erfindungsgemäße Gaswarngerät Teil eines Gerätenetzwerks sein. Dafür verfügt das Gaswarngerät über geeignete, dem Fachmann bekannte Kommunikationselektronik. Die gemessenen Daten können auf dem Gaswarngerät zwischengespeichert werden; dazu hat das Gaswarngerät eine entsprechende Speichereinheit; oder die Daten können direkt weiter an das Netzwerk bzw. einen vorbestimmten Netzwerkknoten oder der Zentraleinheit drahtgebunden oder drahtlos zur weiteren Datenverarbeitung weitergeleitet werden.

Nach einer weiteren erfindungsgemäßen Ausführungsform des Gaswarngeräts weist das Gehäuse eine Schutzhaube auf, die oberhalb des Gehäusedeckels angeordnet ist und den Bereich über der Ausnehmung abdeckt, so dass eine eingesetzte Sensoreinheit vor weiteren Beschädigungen, wie z. B. Stößen geschützt wird. Die Schutzhaube kann aus Stegen oder Platten aufgebaut sein, die sich über den gesamten Gehäusedeckel wölben, und sie hat Durchbrüche. Die Schutzhaube formt eine Brücke, so dass seitlich genug Platz ist, um einen Zugang zu dem Drehring zu ermöglichen.

Eine Gaswarnvorrichtung weist eines oder mehrere erfindungsgemäße Gaswarngeräte und eine datenverarbeitende Zentraleinheit auf. Dabei kann jedes Gaswarngerät mit der Zentraleinheit kommunikativ verbunden werden. Die erfindungsgemäße Gaswarnvorrichtung bildet somit aus einer Vielzahl Gaswarngeräten, die an die Zentraleinheit als zentrale Datenverarbeitungseinheit angeschlossen werden können, ein Mess-Netzwerk, um effektive Gasmessung innerhalb mehrerer Räume oder eines kompletten Gebäudes durchzuführen. Ferner kann eine Gaswarnvorrichtung auch durch die Verbindung der Sensoreinheit, bspw. einem PC als Zentraleinheit, gebildet werden.

Ein erfindungsgemäßes Verfahren zum Durchführen einer Wartungshandlung an dem erfindungsgemäßen Gaswarngerät umfasst die folgenden Schritte:
- Voneinander Lösen der ersten Verriegelungsmittel, die die Ausnehmung aufweist, und der zweiten Verriegelungsmittel, die die Sensoreinheit aufweist, und die lösbar miteinander in Eingriff bringbar sind,
- Entnehmen der Sensoreinheit mit dem Gassensensor aus der Ausnehmung des Gehäuses, und
- Einsetzen einer neuen Sensoreinheit mit einem neuen Gassensor in die Ausnehmung des Gehäuses,
- die ersten Verriegelungsmittel, die die Ausnehmung aufweist, und die zweiten Verriegelungsmittel, die die Sensoreinheit aufweist, und die lösbar miteinander in Eingriff bringbar sind, miteinander in Eingriff bringen.

Es wird vorteilhaft ein einfacher und schneller Sensoraustausch ermöglicht, in dem die komplette Sensoreinheit einfach aus dem Gehäuse herausgenommen bzw. eine neue Sensoreinheit eingesetzt werden kann. Anders als im Stand der Technik erübrigt es sich vorteilhaft, den Sensor bzw. dessen Anschlüsse einzeln elektrisch zu verbinden und aufwändig einzulernen oder das komplette Gaswarngerät austauschen zu müssen. Die Einbettung des Sensors in eine Sensoreinheit mit der für die Sensorik notwendigen Elektronik gibt dem Benutzer die Möglichkeit, einen Sensor auch versatzsicher bzw. positionssicher einzusetzen, so dass weniger Drift in den Messwerten zu erwarten ist, als es bei einem separat einzulernenden und einzubauenden Sensor der Fall wäre.

In einer weiteren Ausführungsform umfasst das erfindungsgemäße Wartungsverfahren, dass zuerst die Drehringposition überprüft wird, wobei die Position des Drehrings je nach Stand in die Austauschposition durch einfaches Drehen des Drehrings gewechselt werden muss. Hiernach werden dann die Verriegelungselemente gelöst, damit die Sensoreinheit herausgenommen werden kann. Um die neu eingesetzte Sensoreinheit dann zu befestigen, wird der Drehring von der Austauschposition zunächst in die Geschlossenposition für eine Testmessung und, wenn der reguläre Messbetrieb aufgenommen werden soll, in die Offenposition gedreht. Sobald der Drehring aus der Austauschposition herausgedreht wird, ist die Sensoreinheit fest und sicher in der Ausnehmung des Gehäuses aufgenommen und gehalten sowie mit den weiteren elektronischen Komponenten, die in dem Gehäuse vorliegen, elektrisch verbunden.

Ein erfindungsgemäßes Verfahren zum Durchführen einer Gasmessung mit dem erfindungsgemäßen Gaswarngerät ermöglicht eine Gasmessung, die ein Testen des Gassensors mit einem Testgas oder ein Messen mit einem Messgas ist. Das Verfahren hat zwei Phasen, wobei in einer ersten Phase, die einer Testphase entspricht, die folgenden Schritte durchgeführt werden:
- In eine Geschlossenposition Bringen des Drehrings in der Ausnehmung, wobei in der Geschlossenposition der Messgas-Fluidpfad von außerhalb der Sensoreinheit bis hin zu dem Gassensor nicht durchgängig ist, wobei die Positionierelemente des Rands der Ausnehmung und die Positionierelemente des Drehrings umfänglich in Bezug zu dem Rand der Ausnehmung zueinander versetzt positioniert sind,
- Zuführen eines Testgases durch den Testgaszugang für das Testgas unter die Abdeckplatte, durch den der Fluidpfad für das Testgas zu dem Gassensor bereitgestellt wird, zu dem Gassensor, wobei der Testgaszugang in der Abdeckplatte der Sensoreinheit vorliegt,
- Erfassen des Testgases mit dem Gassensor und Erzeugen eines Messsignals,
- Ausgeben eines mit dem Messsignal korrelierten Messwertes durch die Steuerungs- und Datenverarbeitungseinheit.

Dadurch, dass während dieser Testmessung der Messgas-Fluidpfad für das Messgas verschlossen wird, ist nur der Testgas-Fluidpfad für das Testgas durchgängig. Vorteilhaft kann das separat über den Testgaszugang direkt in den Messbereich des Gassensors eingebrachte Testgas nicht sofort entweichen, sondern kann dort verweilen. Auf eine zusätzliche Tauchkappe, die über die Sensoreinheit oder das gesamte Gaswarngerät gestülpt wird, um Testgas daran zu hindern, aus dem Messraum um den Gassensor zu entweichen, kann verzichtet werden. Die Reaktionszeit des Gassensors wird dadurch verbessert, so dass nur eine kleine Menge des Testgases benötigt wird. Der Testgaszugang kann bei Nichtgebrauch bspw. mit einem Gummistopfen, der verliersicher an der Abdeckplatte befestigt sein kann, verschlossen werden.

In einer zweiten Phase, die einer Messphase im regulären Betrieb entspricht, werden die folgenden Schritte durchgeführt:
- In eine Offenposition Bringen des Drehrings in der Ausnehmung, wobei in der Offenposition der Messgas-Fluidpfad von außerhalb des Gaswarngeräts bis hin zu dem Gassensor durchgängig ist, und die Positionierelemente des Rands der Ausnehmung und die Positionierelemente des Drehrings teilweise miteinander fluchten, wobei die Positionierelemente des Drehrings die Positionierelemente des Rands der Ausnehmung einander teilweise oder komplett verdecken,
- strömen Lassen eines Messgases entlang dem Messgas-Fluidpfad zu dem Gassensor,
- Erfassen des Messgases mit dem Gassensor und Erzeugen eines Messsignals,
- Ausgeben eines mit dem Messsignal korrelierten Messwertes durch die Steuerungs- und Datenverarbeitungseinheit.

Die erste und die zweite Phase können selbstverständlich auch in umgekehrter Reihenfolge oder in zeitlichem Abstand voneinander ausgeführt werden.

Die Messwerte, die durch die Steuerungs- und Datenverarbeitungseinheit ausgegeben werden, können von der Datenverarbeitungseinheit selbst weiterverarbeitet werden oder mittels der in dem Gehäuse vorliegenden zweiten Kommunikationseinheit an ein Gerätenetzwerk übergeben werden, in dem die Messwerte dann bspw. auf einem geeigneten Netzwerkknoten bzw. der Zentraleinheit weiterverarbeitet werden.

Vorteilhaft wird durch das Drehen des Drehrings der Messgas-Fluidpfad für das Messgas geöffnet oder geschlossen, wobei ein Benutzer durch die Position des Drehrings direkt wahrnehmen kann, in welcher Konfiguration die Sensoreinheit gerade ist und dies leicht ändern bzw. einstellen kann. Es wird dadurch das Einstellen einer bestimmten Betriebskonfiguration, sei es Messbetrieb oder Testbetrieb, einfach und eindeutig.

Der Messgas-Fluidpfad verläuft in einer bevorzugten Ausführungsform des erfindungsgemäßen Gaswarngerätes von außerhalb der Sensoreinheit entlang der sich überlappenden Lücken zwischen den zweiten Platten und der Lücken der Positionierelemente des Randes der Ausnehmung und weiter der sich überlappenden Lücken der dritten Platten und der Lücken der ersten Platten und weiter entlang der Mantelfläche der Ausnehmung über den Steg durch die Durchtrittsöffnungen in der Bodenplatte der Sensoreinheit zu dem Gassensor. Hierdurch strömt das Messgas entlang eines labyrinthartigen Pfades, der einerseits einen Schutz vor Spritzwasser bietet und es andererseits dem Messgas ermöglicht, gleichmäßig den Sensor innerhalb der Sensoreinheit zu umströmen.

Weitere Ausführungsformen des Gaswarngeräts und der Sensoreinheit sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figu-ren sind lediglich eine schematische Darstellung einer Ausführungsform der Erfindung.

Dabei zeigen:
- **Fig. 1**: eine Explosionsansicht eines erfindungsgemäßen Gaswarngeräts,
- **Fig. 2**: eine Explosionsansicht einer erfindungsgemäßen Gaswarnvorrichtung mit Gaswarngerät mit aufgeklappten Gehäusedeckel und schematischer Verbindung zu einer Zentraleinheit,
- **Fig. 3**: eine Explosionsansicht der Sensoreinheit des erfindungsgemäßen Gaswarngeräts,
- **Fig. 4**: eine perspektivische Ansicht der Sensoreinheit und der korrespondierenden Ausnehmung des Gehäuses,
- **Fig. 5**: eine erste Schnittansicht durch das Gaswarngerät mit offenem Drehring,
- **Fig. 6**: eine zweite Teil-Schnittansicht durch das Gaswarngerät mit offenem Drehring und eingezeichnetem Messgas-Fluidpfad b,
- **Fig. 7**: eine weitere Teil-Schnittansicht durch das Gaswarngerät mit geschlossenem Drehring,
- **Fig. 8**: eine noch weitere Schnittansicht durch das Gaswarngerät mit geschlossenem Drehring und eingezeichnetem Testgas-Fluidpfad a,
- **Fig. 9**: eine perspektivische Ansicht auf den Drehring in Kombination mit der Ausnehmung in dem Gehäuse in einer Geschlossenposition,
- **Fig. 10**: eine perspektivische Ansicht auf den Drehring in Kombination mit der Ausnehmung in dem Gehäuse in einer Austauschposition,
- **Fig. 11**: a) eine Draufsicht, b) eine Seitenansicht und c) eine Schnittansicht entlang B-B des Drehrings in Geschlossenposition,
- **Fig. 12**: a) eine Draufsicht, b) eine Seitenansicht und c) eine Schnittansicht entlang A-A des Drehrings in Offenposition,
- **Fig. 13**: eine Explosionsansicht einer weiteren Ausführungsform des Gaswarngeräts,
- **Fig. 14**: eine Explosionsansicht der weiteren Ausführungsform der Gaswarnvorrichtung, und
- **Fig. 15**: eine Schnittansicht durch die weitere Ausführungsform des Gaswarngeräts mit offenem Drehring.

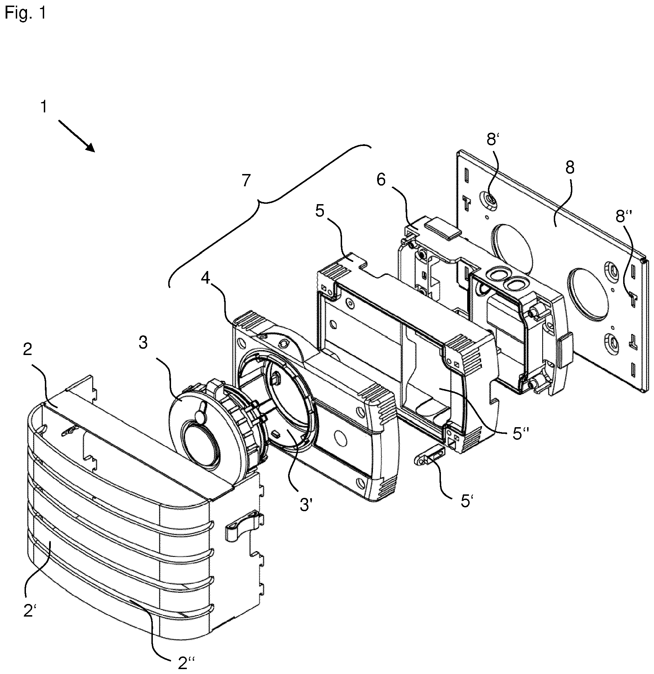

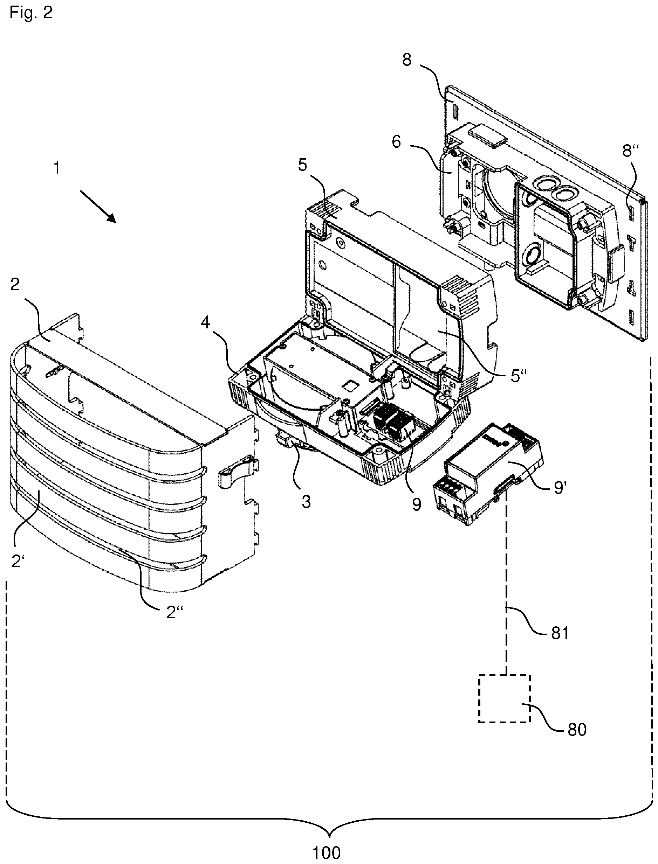

In **Fig. 1** und **2** ist ein Gaswarngerät 1 gezeigt, das eine Schutzhaube 2, eine Sensoreinheit 3 und ein Gehäuse 7 aufweist, wobei das Gehäuse 7 auf einer Montageplatte 8 befestigt wird, die dafür Öffnungen 8' hat. Die Montageplatte 8 dient zur Montage des Gehäuses 7 des Gaswarngeräts 1 bspw. an einer Wand und weist dafür notwendige Befestigungsöffnungen 8" auf.

Das Gehäuse 7 weist einen Gehäusedeckel 4 mit einer Ausnehmung 3` auf, in der die Sensoreinheit 3 aufgenommen werden kann und die geometrisch korrespondierend zu der Sensoreinheit 3 geformt ist.

Der Gehäusedeckel 4 ist an einem Gehäusekörper 5 über Scharniere 5' angelenkt, wobei der Gehäusekörper 5 auf einem Gehäuseunterteil 6 aufsitzt. Das Gehäuseunterteil 6 ist in einer Montageanordnung mit der Montageplatte 8 verschraubt, wie insbesondere **Fig. 2** zeigt. Der Gehäusekörper 5 nimmt in einer Ausnehmung 5" ein Gehäuse 9' mit Kommunikationselektronik 9 auf, die u. a. eine Netzwerkschnittstelle für eine drahtlose oder drahtgebundene Verbindung 81 mit einer Zentraleinheit 80 (in **Fig. 2** beides gestrichelt dargestellt) aufweist. Zusammen mit der Zentraleinheit 80 bildet das Gaswarngerät 1 eine Gaswarnvorrichtung 100.

Die Schutzhaube 2 weist eine Vielzahl parallel zueinander angeordnete gebogene Stege 2` auf, die die darunterliegende Sensoreinheit 3 überbrücken und Schutz vor Beschädigungen bieten. Zwischen den Stegen 2' sind Durchbrüche 2", die die Stege 2' voneinander beabstanden.

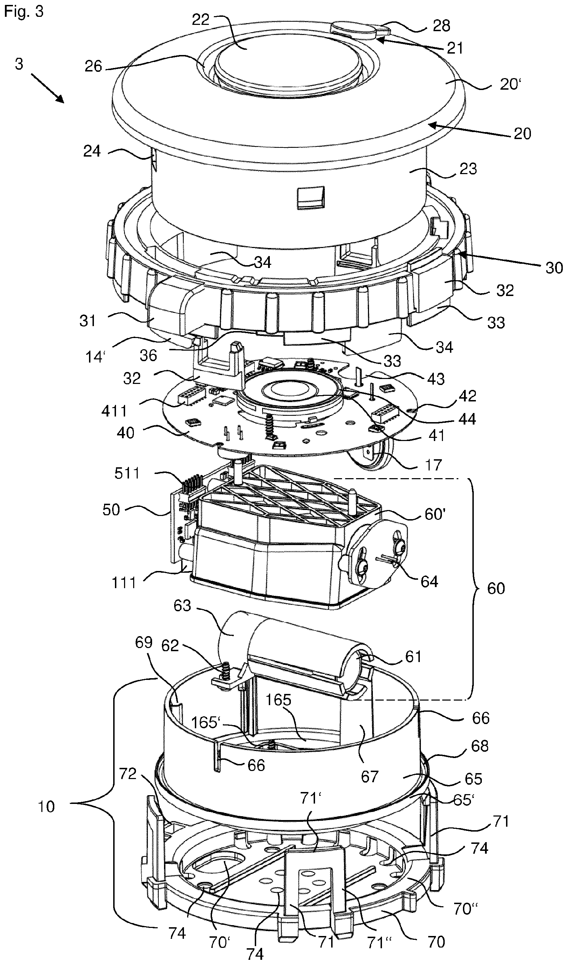

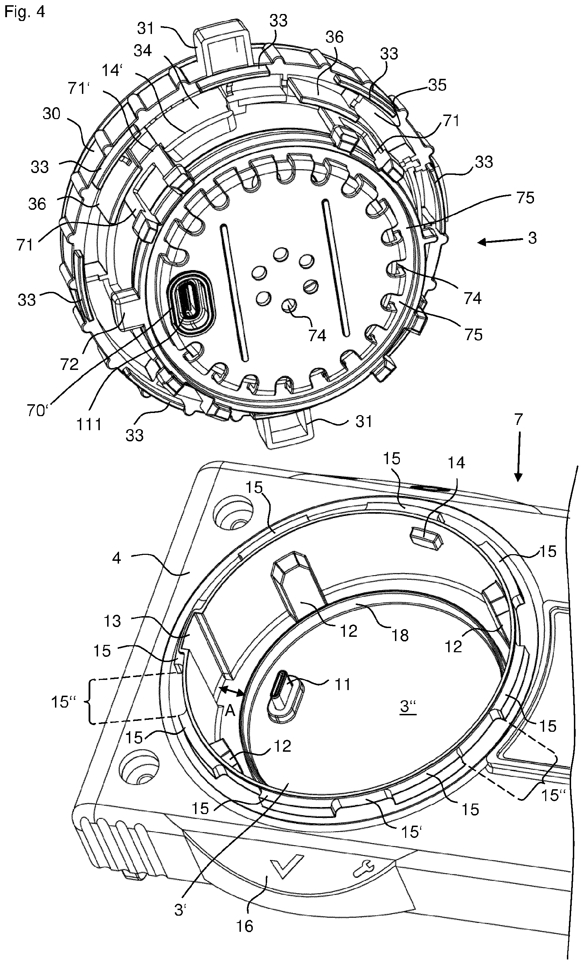

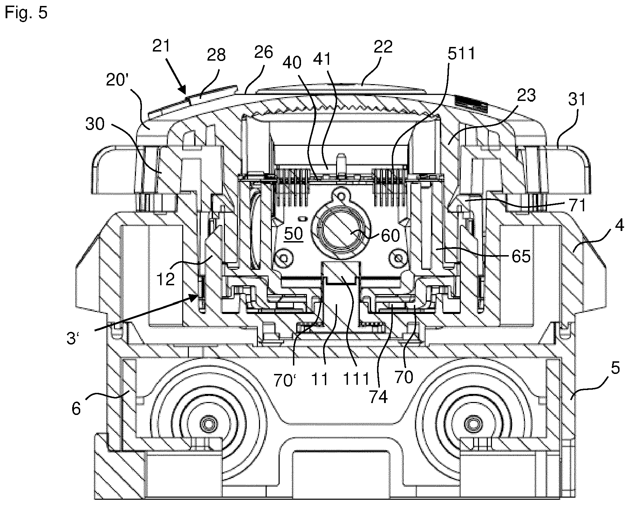

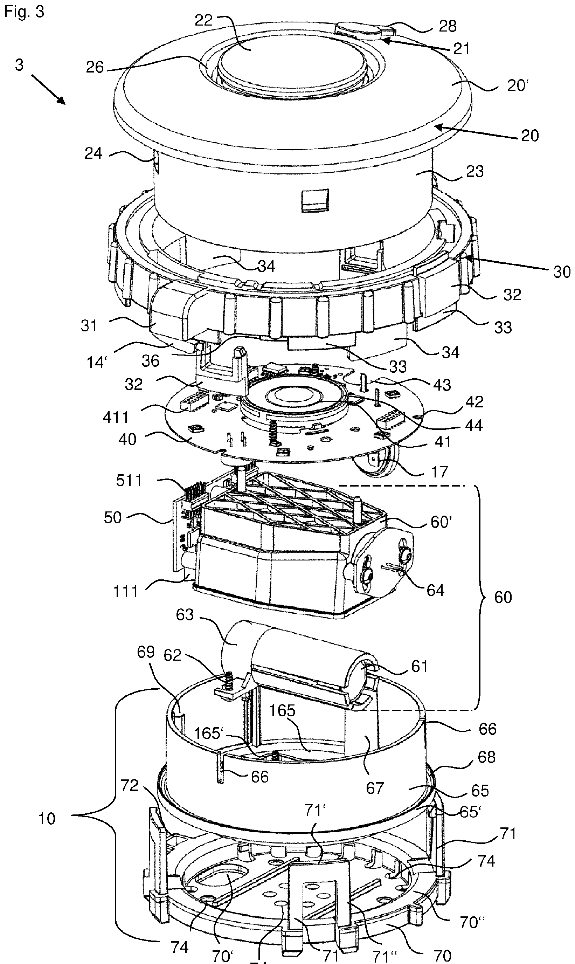

Die Sensoreinheit 3 und ihr Einsatz in den Gehäusedeckel 4 des Gehäuses 7 sind im Detail in **Fig. 3** und **4** gezeigt sowie in Schnittdarstellungen in **Fig. 5 bis 8****,** wobei die Sensoreinheit 3 als wesentliche Bauteile eine kreiszylindrische Aufnahmevorrichtung 10, eine Sensorplatine 50 mit angeschlossenem Gassensor 60 und einen Deckel 20 aufweist. Der Gassensor 60 weist ein Messrohr 61 in einer Messrohrwanne 60' auf, wobei das Messrohr 61 in einer Messrohrhalterung 63 angeordnet ist und über eine Verschraubung 62 in der Aufnahmevorrichtung 10 befestigt ist. Die Messrohrwanne 60' haust das Messrohr 61 ein und hält an einer ihrer Außenseiten die Sensorplatine 50, sowie an der von der Sensorplatine 50 abgewandten Seite eine Lichtquelle 64, die die Messstrecke, deren Länge durch die Abmessung des Messrohrs 61 bestimmt wird, durchleuchten kann. Die Sensorplatine 50 weist geeignete Erfassungsmittel für das von der Lichtquelle 64 ausgesandte Licht im IR- oder UV-Bereich, die sich für die Detektion von CO₂ eignet.

Die Aufnahmevorrichtung 10 hat eine kreisförmige Grundplatte 70, die außenumfänglich vier Haltebügel 71 als Haltemittel (**Fig. 3**) hat. Ferner ist in der Grundplatte 70 eine Ausnehmung 70' vorgesehen, die einen Durchtritt für eine elektronische Schnittstelle 11 bildet, die in der Ausnehmung 3' am Boden 3" des Gehäusedeckels 4 des Gehäuses 7 vorliegt, wie in **Fig. 4** und **Fig. 5** dargestellt. Ferner weist die Grundplatte 70 eine Vielzahl Durchtrittsöffnungen 74 entlang ihres Umfangs und in der Mitte auf, die ein Messgas durchtreten lassen. Ein verdickter Rand 70" der Grundplatte 70 bildet eine Umrandung für die Durchtrittsöffnungen 74 und weist einen Vorsprung 72 auf, der als Orientierungshilfe dient, wenn die Sensoreinheit 3 in die Ausnehmung 3' in dem Gehäusedeckel 4 eingesetzt werden soll.

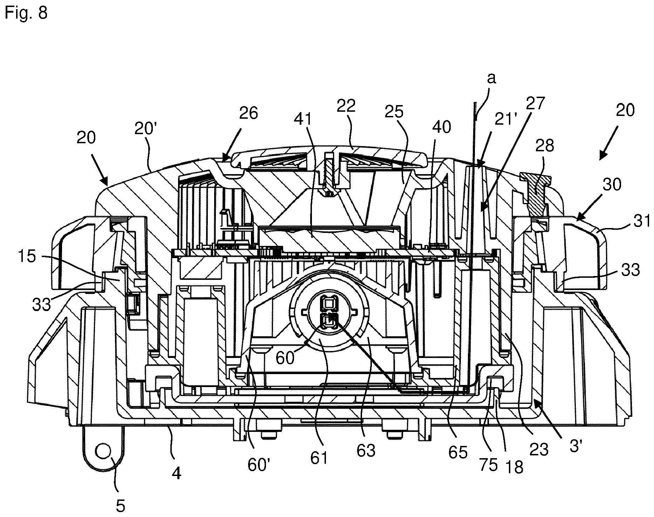

Die Aufnahmevorrichtung 10 weist ferner ein Ringelement 65 mit einem Bodenabschnitt 165 auf, in dem eine Ausnehmung 165` vorliegt, die mit den Durchtrittsöffnungen 74 der Grundplatte 70 fluidisch verbunden ist. Das Ringelement 65 weist einen außenumfänglichen Flansch 65' auf, der an der dem Bodenabschnitt 165 zugewandten Kante des Ringelements 65 vorliegt. In dem Flansch 65' ist eine Nut oder Dichtmittelaufnahme 68 gebildet, in die ein Dichtmittel eingelegt werden kann. Das Ringelement 65 weist ferner an seiner dem Bodenabschnitt 165 abgewandten (in **Fig. 3**) oberen Kante Schlitze 66 auf, die zur Befestigung einer weiteren Platine, der Prozessorplatine 40, die sich auf der oberen Kante abstützt, dienen. Die Prozessorplatine 40 weist dazu korrespondierende Schlitze 42 auf, in die geeignete Befestigungselemente eingreifen können. Das Ringelement 65 dient als Einfassung für den Gassensor 60. Die Verschraubung 62 der Messrohrhalterung 63 ist an dem Bodenabschnitt 165 des Ringelements 65 befestigt. Die Prozessorplatine 40 trägt einen Lautsprecher 41, der über eine ringförmige Dichtung 44 nach oben mit einem Konus 25 des Deckels 20 (vgl. **Fig. 8**) von innen abdichtet.

Auf der Sensorplatine 50 sind verschiedene elektronische Komponenten angeordnet, die der Steuerung des Gassensors 60, der Erfassung von Messdaten und deren Datenverarbeitung dienen. Die Elektronik der Sensorplatine 50 bildet die Steuerungs- und Datenverarbeitungseinheit der Sensoreinheit 3 und ermöglicht eine komplette Datenauswertung auf der Sensoreinheit. Die Prozessorplatine 40 kann weitere elektronische Komponenten tragen, die für die Datenverarbeitung notwendig sind. So ist an der Prozessorplatine 40 eine Batterie 17 befestigt, die eine Uhr mit Kalender betreibt, die die Zeit misst, wie lange die Sensoreinheit 3 aktiv ist.

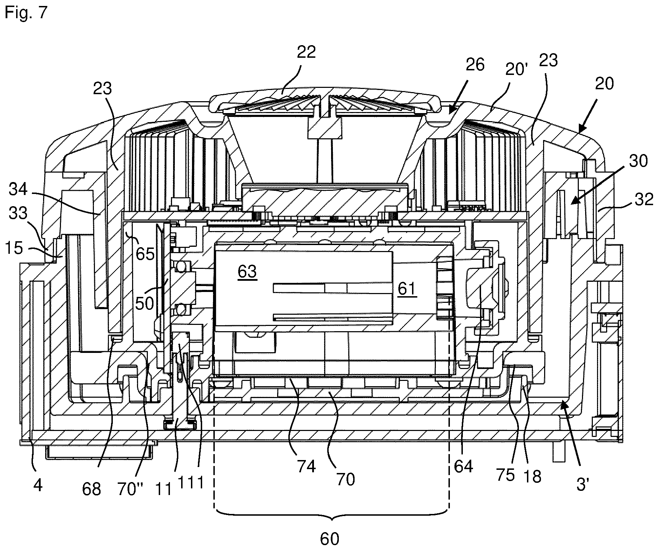

Die Sensorplatine 50 trägt ferner eine dem Aufnahmeelement 10 zugewandte elektronische Schnittstelle 111 und eine der Prozessorplatine 40 zugewandte elektronische Schnittstelle 511. Die Prozessorplatine 40 weist eine elektronische Schnittstelle 411 auf, die mit der elektronischen Schnittstelle 511 der Sensorplatine 50 korrespondiert. Sie hat einen U-förmigen Ausschnitt 43 am Rand. Die genannten elektronischen Schnittstellen 111, 511, 411 sind in der Betriebsanordnung elektrisch und elektronisch verbunden, wobei die dem Aufnahmeelement 10 zugewandte elektronische Schnittstelle 111 mit der elektronischen Schnittstelle 11, die durch die Ausnehmung 70' in der Grundplatte 70 tritt, korrespondiert und elektrisch und elektronisch verbunden ist, wie in **Fig. 5** und **7** gezeigt ist.

Die insgesamt aufgezählten elektronischen Schnittstellen 511, 411, 111 und 11 stehen, wie **Fig. 3 bis 7** verdeutlichen, in einer Betriebsanordnung des Gaswarngeräts 1 in direktem elektrischem Kontakt, bei dem Sensoreinheit 3 und Gehäuse 7 miteinander mechanisch verbunden sind. Es wird hiermit der elektrische und elektronische Kontakt zwischen der Elektronik für den Gassensor 60, der mit der Sensorplatine 50 verbunden ist, und der Elektronik für eine Kommunikation mit der Zentraleinheit 80 bereitgestellt, wobei diese Elektronik im Gehäuse 7 angeordnet ist.

Das Ringelement 65 weist eine Rinne 67 für den Testgaszugang und Schienenelemente 69 auf, die eine Führung für die Sensorplatine 50 bieten und diese in einer Betriebsanordnung positionssicher halten.

Der Deckel 20 ist pilzförmig und weist eine Abdeckplatte 20' sowie einen Einsatz 22 auf. Der Deckel 20 weist ferner einen kreiszylindrischen Mantel 23 auf, der sich gegen den Flansch 65` abstützt und die Abdeckplatte 20' trägt. An dem kreiszylindrischen Mantel 23 sind in der Mitte umfänglich gleichverteilt insgesamt vier Rastausnehmungen 24, die mit den Haltebügeln 71 einrasten.

Die Abdeckplatte 20' weist ferner eine Testzugang 21 auf, mit dem Testgas über einen Fluidpfad a direkt an den Gassensor 60 geleitet werden kann. Wie in **Fig. 8** ersichtlich, kann ein Fluidpfad a (Testgas-Fluidpfad) von eingebrachtem Testgas durchströmt werden.

Zwischen dem kreisförmigen Einsatz 22 und der Abdeckplatte 20' ist ein Spalt 26 gebildet, der als Austrittsspalt für akustische Signale des Lautsprechers 41 dient. Hierzu weist die Abdeckplatte 20' ferner einen inneren Konus 25 auf, der einen Luftraum oberhalb des Lautsprechers 41 begrenzt.

Unterhalb der Abdeckplatte 20' angeordnet und überkragt von dieser liegt um den Mantel 23 ein Drehring 30 vor, der um den Mantel 23 drehbar gelagert ist. Der Drehring 30 stützt sich mit einer innenumfänglichen Unterkante, wie **Fig. 5** zeigt, die der Grundplatte 70 zugewandt ist, auf eine Oberkante 71' der Haltebügel 71 der Grundplatte 70 ab. Die Drehbewegung kann in zwei, maximal drei Positionen erfolgen, wobei die Drehbewegung des Drehrings 30 durch Anlage von Positionierelementen 34 an Seitenkanten 71" der Haltebügel 71 begrenzt wird, siehe dazu **Fig. 4****.**

Der Drehring 30 weist drei Typen Positionierelemente auf, wie die **Fig. 3****,** **4****,** **9, 10****,** **11 c)** und **12c)** zeigen. Diese Positionierelemente bewirken im Zusammenspiel mit Positionierelementen 15, die an einem Rand 15' der Ausnehmung 3' vorliegen, dass der Drehring 30 verschiedene Positionen einnehmen kann, wie weiter unten genauer ausgeführt wird.

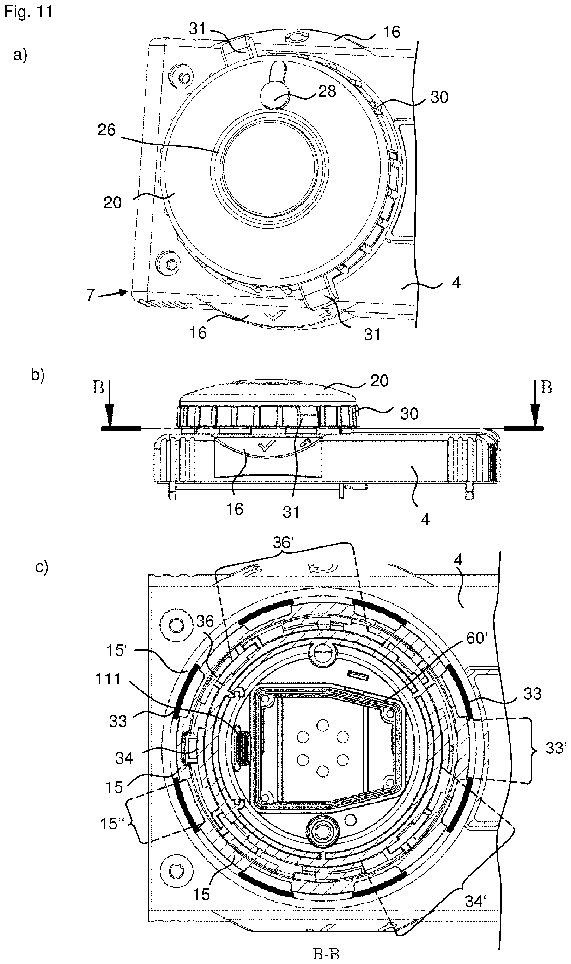

Die Positionierelemente aller Typen sind erste Platten 34, zweite Platten 33 und dritte Platten 36, die sich von einer zu der Grundplatte 70 weisenden Unterseite des Drehrings 30 in Richtung der Grundplatte 70 erstrecken. Die Platten 33, 34, 36 liegen auf gedachten Kreisringen mit unterschiedlichen Radien. Die ersten Platten 34 sind radial näher zu einem Mittelpunkt des Drehringes 30 an dessen Unterseite des Drehrings 30 angeordnet und liegen am inneren Umfang des Drehrings 30 vor. Sie sind durch Lücken 34' voneinander beabstandet, wie **Fig. 11c****)** und **12c****)** zeigen. Die zweiten Platten 33 sind ebenfalls an der Unterseite des Drehrings 30, aber an dessen äußerem Umfang angeordnet und sind ebenfalls durch Lücken 33' voneinander beabstandet. Die Lücken 34' zwischen den ersten Platten 34 und die Lücken 33' zwischen den zweiten Platten 33 sind nicht kongruent zueinander und in Bezug auf eine axiale Erstreckung der Platten 33, 34 sind die zweiten Platten 33 kürzer als die ersten Platten 34.

Die dritten Platten 36 erstrecken sich von einer zu der Grundplatte 70 weisenden Unterseite des Drehrings 30 in Richtung der Grundplatte 70, wobei die dritten Platten 36 in Bezug auf die radiale Anordnung zu einem Mittelpunkt des Drehringes 30, d. h. auf einer Kreisumfangslinie zwischen den ersten Platten 34 und den zweiten Platten 33 ebenfalls an der Unterseite des Drehrings 30 angeordnet sind. Auch die dritten Platten 36 sind durch Lücken 36' voneinander beabstandet angeordnet. Dabei sind die Lücken 34' der ersten Platten 34 und die Lücken 33` der zweiten Platten 33 nicht kongruent mit den Lücken 36` der dritten Platten 36. In Bezug auf eine axiale Erstreckung der dritten Platten 36 sind die dritten Platten 36 kürzer als die ersten Platten 34 und gleich lang wie die zweiten Platten 33.

Die Ausnehmung 3' in dem Gehäusedeckel 4 des Gehäuses 7 ist im Wesentlichen kreiszylindrisch, wie in **Fig. 4** gezeigt ist, und weist einen über eine Oberfläche des Gehäusedeckels 4 überkragenden Rand 15' mit umfänglich äquidistant zueinander verteilten, nach außenweisenden Positionierelementen 15 auf, zwischen den Lücken 15" vorliegen. Die Lücken 15" sind dabei genauso lang wie die Positionierelemente 15.

Ferner hat die Ausnehmung 3` einen Boden 3". Auf dem Boden 3" sind an einer inneren Mantelfläche der Ausnehmung 3' des Gehäuses 7 verteilt Haltestege 12 angeordnet, die längsaxial in Bezug zu der Mantelfläche der Ausnehmung 3' verlaufen. Ein Einsetzen der Sensoreinheit 3 in die Ausnehmung 3' des Gehäuses 7 beinhaltet, dass der Vorsprung 72 in die Rinne 13 geführt wird und die Sensoreinheit 3 senkrecht in die Ausnehmung 3' geführt wird. Damit ist ein sicheres Einsetzen möglich, so dass die Sensoreinheit 3 sich nicht verkantet und auch die elektronischen Schnittstellen 111 und 11 korrekt verbunden und nicht beschädigt werden. Die Haltestege 12 sind so beschaffen, dass sie mit den Haltebügeln 71 an der kreiszylindrischen Grundplatte 70 der Sensoreinheit 3 zum lösbaren Halten derselben in der Ausnehmung 3' in Eingriff stehen können. Die Haltebügel 71 werden einfach über die Haltestege 12 geschoben.

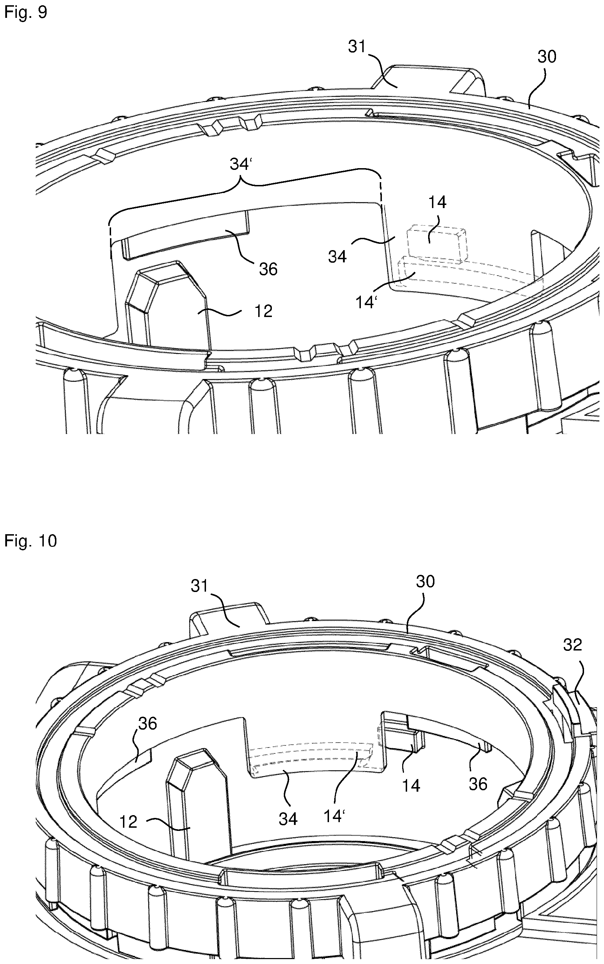

Damit die Sensoreinheit 3 in der Ausnehmung 3' verriegelt gehalten werden kann, und um zu verhindern, dass die Sensoreinheit 3 herausfällt, wenn der Drehring 30 bedient wird, sind an Sensoreinheit 3 und Ausnehmung 3' zueinander korrespondierende Verriegelungselemente 14, 14' vorgesehen. Die Ausnehmung 3' weist erste Verriegelungselemente, nämlich zwei an der inneren Mantelfläche der Ausnehmung 3' vorliegende und radial nach innen weisende Nasen 14 auf (von denen in **Fig. 4****,** **9** und **10** jeweils nur eine zu sehen ist). Die Sensoreinheit 3, bzw. der Drehring 30 weist zweite Verriegelungsmittel auf, die an der Außenseite zweier erster Platten 34 sitzen und nach außen weisende, zur Mantelfläche der Ausnehmung 3` gewandte Längsstege 14' sind (stricheliert in **Fig. 9** und **10**). In der Betriebsanordnung untergreifen die Längsstege 14' die nach innen weisenden Nasen 14. Die Verriegelungsmittel können über Entriegelungselemente 32 gelöst werden, die als Federelemente mit Kugelelementen ausgebildet sind (siehe **Fig. 3** und **7**).

Ist die Sensoreinheit 3 in der Ausnehmung 3' aufgenommen, umgreift der Drehring 30 mit seinen Positionierelementen (Platten 33, 34, 36) den Rand 15' der Ausnehmung 3'. Es wird ein Umgriff 35 gebildet, der den Rand 15' umfasst, wie auch **Fig. 4** andeutet.

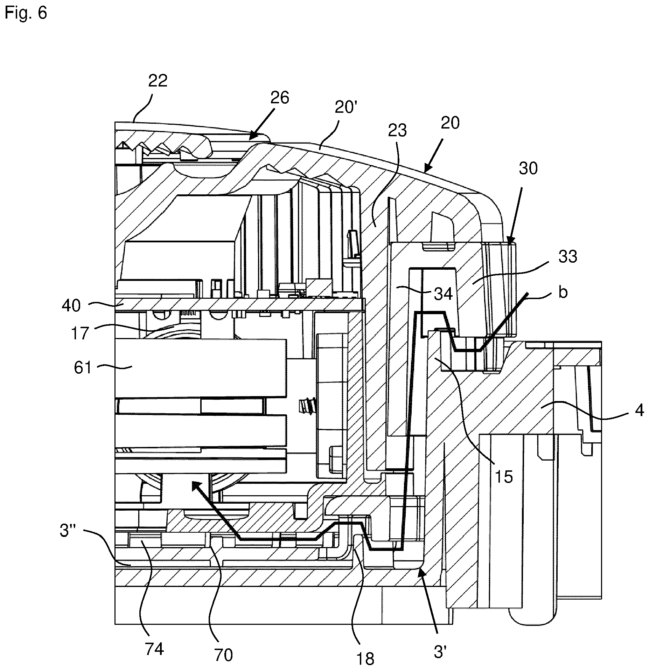

Ferner liegt auf dem Boden 3" der Ausnehmung 3' in dem Gehäusedeckel 4 ein kreisförmiger Steg 18 vor, der mit einem Abstand A von der inneren Mantelfläche der Ausnehmung 3' beabstandet ist. Dazu korrespondierend weist die Grundplatte 70 der Sensoreinheit 3 an ihrer zu dem Boden 3" weisenden Seite umfangsnah eine Nut 75 auf, wobei die Nut 75 den kreisförmigen Steg 18 aufnimmt, aber nicht komplett abschließt. Damit wird ein Teil des Fluidpfades b (Messgas-Fluidpfad, siehe **Fig. 6**) bereitgestellt, indem vor allem die Nut 75 fluidisch mit zumindest einem Teil der Durchtrittsöffnungen 74 der Grundplatte 70 kommuniziert. Ein weiterer Teil des Fluidpfades b wird durch den Abstand zwischen Mantelfläche der Ausnehmung 3' und der äußeren Mantelfläche des Mantels 23 des Deckels 20 gebildet.

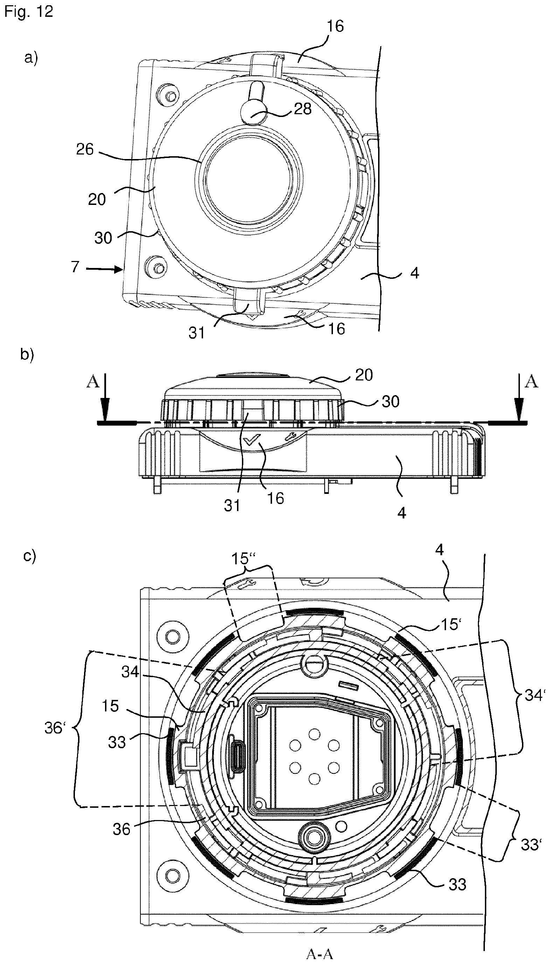

Der Drehring 30 bildet im Zusammenspiel mit dem Rand 15' einen weiteren wesentlichen Teil des Fluidpfades b, wie im Weiteren erläutert wird. Der Drehring 30 hat zwei Griffe 31, die sich an einem äußeren Umfang des Drehrings 30 gegenüberliegen. Mit dem Griff 31 kann der Drehring 30 in die verschiedenen Positionen verstellt werden, wie sie auch in **Fig. 11** und **12** jeweils **a) bis c)** gezeigt sind. Die Stellung des Griffs 31 ermöglicht ferner anzuzeigen, in welcher Position sich der Drehring befindet. Dazu weist der Gehäusedeckel 4 an geeigneter Stelle eine Anzeige 16 (siehe dazu **Fig. 4**) auf, die in das Material des Gehäuses eingebrachte Piktogramme hat, so dass ein Drehen des Drehrings 30 und Einstellen des Griffs 31 auf die Position entsprechend einem Piktogramm sofort der Modus der Sensoreinheit 3 klar ersichtlich ist. In **Fig. 11a****)** und **b)** ist der Drehring 30 in einer Geschlossenposition (entsprechend einer Testkonfiguration, Piktogramm zeigt einen stilisierten Schraubenschlüssel) und in **Fig. 12a****)** und **c)** ist der Drehring 30 in einer Offenposition (entsprechend einer Messkonfiguration, Piktogramm zeigt einen OK-Haken).

In den **Fig. 6 bis 10****,** **11c)** und **12c)** ist dargestellt, wie sich die Anordnung der unterschiedlichen Positionierelemente von Drehring 30 und Gehäuse 7 zueinander verändert, wenn der Drehring 30 die verschiedenen Positionen einnimmt. Für den Fluidpfad b, der Messgas zu dem Gassensor 60 strömen lassen soll, kann der Drehring 30 eben in die Geschlossenposition oder in die Offenposition gebracht werden.

In **Fig. 7****,** **8** und **11c****)** ist die Geschlossenposition gezeigt, in der der Fluidpfad b von außerhalb des Gaswarngeräts 1 bis hin zu dem Gassensor 60 nicht durchgängig ist, wie es für eine Testmessung der Fall ist. Dabei sind die Positionierelemente 15 und die zweiten Platten 33 umfänglich in Bezug zu dem Rand 15' der Ausnehmung 3' zueinander versetzt positioniert. Die Lücken 33', 15" zwischen den zweiten Platten 33 und den Positionierelementen 15 fluchten nicht. Weil beide Lücken 33', 15" gleiche Abmessungen haben, wird der Zugang für Messgas verschlossen. Die dazu versetzt angeordneten ersten Platten 34 und dritten Platten 36 dienen als weitere Verschlusselemente innerhalb der Ausnehmung 3'.

In dieser Position lässt sich über den Testgaszugang 21 Testgas von außerhalb des Gaswarngeräts 1 in die Sensoreinheit 3 einbringen. Das Testgas kann ausgehend von dem Testgaszugang 21, der einer mit einem Stopfen 28 verschließbaren Öffnung 21' entspricht, durch einen Zugangskanal 27, der von der Oberfläche der Abdeckplatte 20' ins Innere des Deckels 20 führt, durch die Rinne 67 entlang der inneren Mantelfläche des Ringelements 65 der Aufnahmevorrichtung 10, entlang der Innenfläche der Grundplatte 70 der Aufnahmevorrichtung 10 durch die Ausnehmung 165' bis hin zu dem Gassensor 60 strömen. Der Stopfen 28 ist in **Fig. 8** nur teilweise gezeigt und ist in dem Deckel 20 verankert.

In **Fig. 6** und **12c****)** ist die Offenposition des Drehrings 30 gezeigt, die in der Betriebsanordnung des Gaswarngeräts 1 einer üblichen Messsituation entspricht. Der Fluidpfad b ist offen und lässt Messgas von außerhalb des Gaswarngeräts 1 bis zum Gassensor 60 strömen. Dabei fluchten die zweiten Platten 33 mit den Positionierelementen 15, so dass die Lücken 33', 15" zwischen den zweiten Platten 33 und den Positionierelementen 15 ebenfalls fluchten. Die weiteren Lücken 36' zwischen den dritten Platten 36 und die Lücken 34` zwischen den ersten Platten 34 überlappen sich und stellen einen weiteren Teil des Fluidpfades b bereit.

Der Fluidpfad b, dargestellt als schwarzer Pfeil in **Fig. 6****,** verläuft von außerhalb des Gaswarngeräts 1 entlang der sich überlappenden Lücken 33' der zweiten Platten 33 und der Lücken 15" der Positionierelemente 15 und weiter entlang der sich überlappenden Lücken 36' zwischen den dritten Platten 36 und der Lücken 34' zwischen den ersten Platten 34 und weiter entlang der Mantelfläche der Ausnehmung 3' über den Steg 18 durch die Nut 75 durch die Durchtrittsöffnungen 74 bis hin zu dem Gassensor 60.

Ferner kann der Drehring 30 eine Austauschposition einnehmen, wie es in **Fig. 10** dargestellt, in der die Verriegelungselemente Nasen 14 und Längsstreben 14' nicht in Eingriff miteinander stehen. Der Drehring 30 wird dazu etwas weiter als die Geschlossenposition gedreht und die Verriegelungselemente manuell gelöst, so dass die gesamte Sensoreinheit 3 aus der Ausnehmung 3' herausgenommen werden kann.

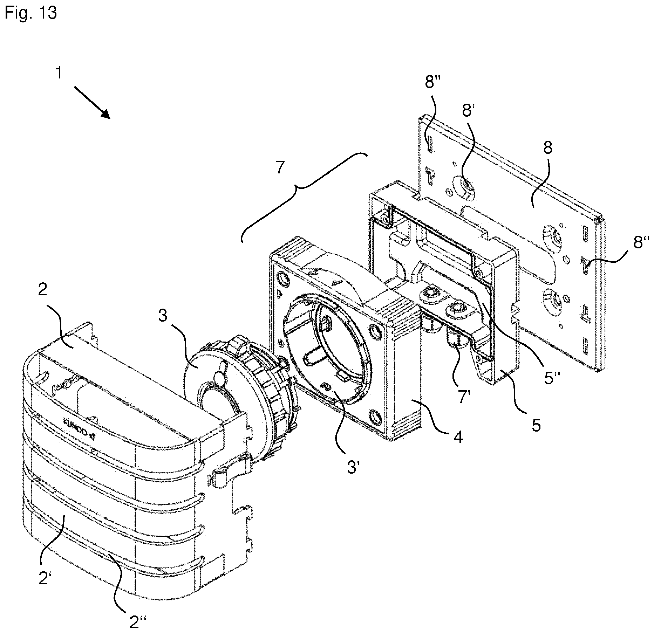

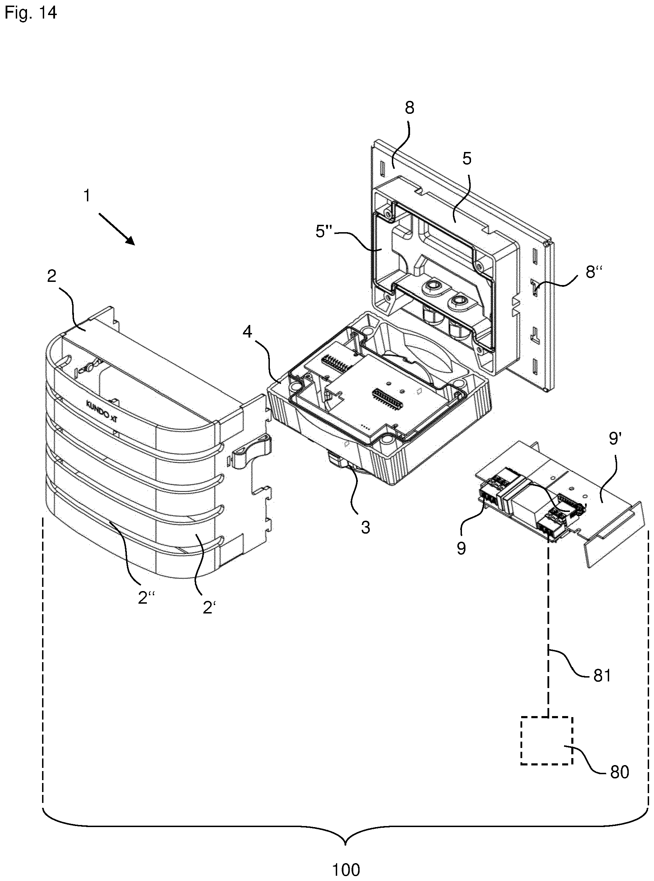

In den **Fig. 13****,** **14** und **15** ist ein weiteres Gehäuse 7 für das Gaswarngerät 1 gezeigt, wobei der generelle Aufbau des dort gezeigten Gaswarngeräts 1 dem des in **Fig. 1 bis 3** dargestellten Gaswarngeräts 1 entspricht. Das Gehäuse 7 des in **Fig. 13****,** **14** und **15** dargestellten Gaswarngeräts 1 ist kompakt gestaltet und kommt ohne Scharniere oder weitere zusätzliche Verbindungselemente aus. Der Gehäusedeckel 4 weist die Ausnehmung 3' auf, in der die Sensoreinheit 3 eingesetzt werden kann. Der darunter liegende Gehäusekörper 5, in dessen Ausnehmung 5" das Gehäuse 9' der Kommunikationselektronik 9 aufgenommen ist, kann mit dem Gehäusedeckel 4 verschraubt bzw. zusammengesteckt werden. In dem Gehäusekörper 5 sind Anschlussbuchsen 7' angeordnet, mittels denen die Kommunikationselektronik 9 über die elektrische Verbindung 81 mit der Zentraleinheit 80 verbunden werden kann (in **Fig. 14** beides gestrichelt dargestellt). Zusammen mit der Zentraleinheit 80 bildet das Gaswarngerät 1 die Gaswarnvorrichtung 100.

Das Gehäuse 7 wird auf der Montageplatte 8 befestigt, die hierfür Öffnungen 8' hat, um den Gehäusekörper 5 mit der Montageplatte 8 zu verschrauben. Die Montageplatte 8 weist Befestigungsöffnungen 8" auf, die zur Montage des Gehäuses 7 des Gaswarngeräts 1 bspw. an einer Wand dienen.

Über dem Gehäusedeckel 4 wird die Schutzhaube 2 aufgesetzt, die eine Vielzahl parallel zueinander angeordnete gebogene Stege 2' aufweist, die die darunterliegende Sensoreinheit 3 überbrücken und Schutz vor Beschädigungen bieten. Zwischen den Stegen 2` sind wie in der **Fig.1** und **2** schon gezeigt Durchbrüche 2", die die Stege 2' voneinander beabstanden.

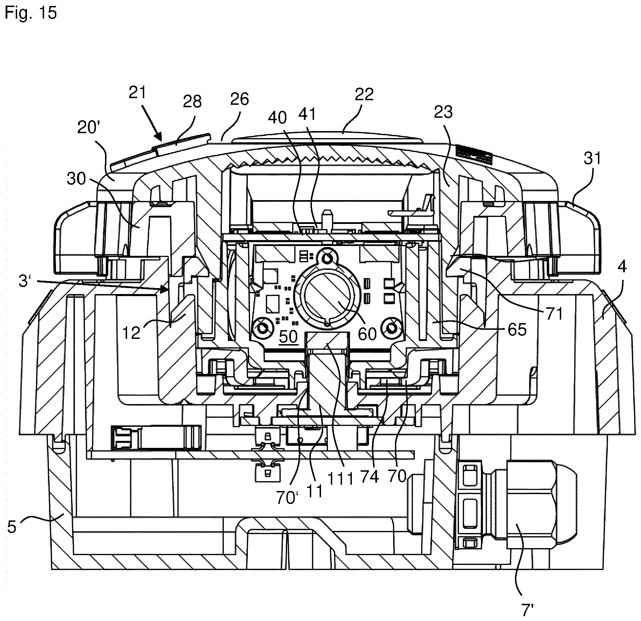

In **Fig. 15** zeigt das Gaswarngerät 1 im gleichen Schnitt wie Fig. 5, wobei die Sensoreinheit 3 in der Ausnehmung 3' des Gehäusedeckels 4 eingesetzt ist und die elektronische Schnittstelle 11 der Sensoreinheit mit der elektronischen Schnittstelle 111 der Ausnehmung 3' des Gehäusedeckels 4 verbunden ist. Der Gehäusedeckel 4 sitzt in dieser Ausführungsform direkt auf dem Gehäusekörper 5 auf, der auch die Anschlussbuchsen 7' trägt. Die weiteren Komponenten entsprechen der obigen Beschreibung zu **Fig. 5****.**

### BEZUGSZEICHENLISTE

- 1: Gaswarngerät
- 2: Schutzhaube
- 2': gebogene Stege
- 2": Durchbrüche
- 3: Sensoreinheit
- 3': korrespondierende Ausnehmung
- 3": Boden
- 4: Gehäusedeckel
- 5: Gehäusekörper
- 5': Scharniere
- 5": Ausnehmung
- 6: Gehäuseunterteil
- 7: Gehäuse
- 7': Anschlussbuchsen
- 8: Montageplatte
- 8': Öffnungen
- 8": Öffnungen
- 9: Kommunikationselektronik
- 9': Gehäuse Kommunikationselektronik
- 10: Aufnahmevorrichtung
- 11: elektronische Schnittstelle Ausnehmung
- 111: elektronische Schnittstelle Sensoreinheit
- 12: Haltestege
- 13: Rinne
- 14: Nase
- 14': Längsstege
- 15: Positionierelement
- 15': Rand der Ausnehmung
- 15": Lücke zwischen Positionierelementen
- 16: Anzeige
- 17: Batterie
- 18: Steg
- 20: Deckel
- 20': Abdeckplatte
- 21: Testgaszugang
- 21': Öffnung
- 22: Einsatz
- 23: kreiszylindrischer Mantel (des Deckels 20)
- 24: Rastausnehmung
- 25: Konus
- 26: Spalt für Lautsprecher
- 27: Zugangskanal
- 28: Stopfen
- 30: Drehring
- 31: Griff
- 32: Entriegelungselement
- 33: Positionierelement/zweite Platte
- 33': Lücke zwischen zweiten Platten
- 34: Positionierelement/erste Platten
- 34': Lücke zwischen ersten Platten
- 35: Umgriff
- 36: Positionierelement/dritte Platten
- 36': Lücke zwischen dritten Platten
- 40: Prozessorplatine
- 41: Lautsprecher
- 42: Schlitze
- 43: U-förmiger Ausschnitt
- 44: Dichtung Lautsprecher
- 50: Sensorplatine/Steuerungs- und Datenverarbeitungseinheit
- 60: Gassensor
- 60': Messrohrwanne
- 61: Messrohr
- 62: Verschraubung
- 63: Messrohrhalterung
- 64: Lichtquelle
- 65: Ringelement
- 65`: Flansch
- 66: Schlitz
- 67: Rinne
- 68: Nut/Dichtmittelaufnahme
- 69: Führung Sensorplatine
- 70: Grundplatte
- 70': Ausnehmung elektronische Schnittstelle
- 70": verdickter Rand
- 71: Haltebügel
- 71': obere Kante
- 71": Seitenkante
- 72: Vorsprung
- 74: Durchtrittsöffnungen
- 75: Nut
- 80: Zentraleinheit
- 81: elektrische Verbindung
- 100: Gaswarnvorrichtung
- 165: Bodenabschnitt des Ringelements
- 165`: Ausnehmung
- 411: elektronische Schnittstelle Prozessorplatine
- 511: elektronische Schnittstelle Sensorplatine

- A: Abstand
- a: Fluidpfad Testgas
- b: Fluidpfad Messgas

## Patentansprüche

1. Gaswarngerät (1) das eine lösbar darin angeordnete Sensoreinheit (3) aufweist, **wobei** das Gaswarngerät (1)
ein Gehäuse (7) mit einem Gehäusedeckel (4), einem Gehäusekörper (5) und einem Gehäuseunterteil (6) aufweist, wobei in dem Gehäusedeckel (4) eine kreiszylindrische Ausnehmung (3') mit einem Boden (3") vorliegt, der eine elektronische Schnittstelle (11) aufweist, und wobei
die Sensoreinheit (3) eine kreiszylindrische Aufnahmevorrichtung (10) hat, in der ein Gassensor (60) und zumindest eine Steuerungs- und Datenverarbeitungseinheit (50), die in kommunikativer Verbindung mit dem Gassensor (60) steht, angeordnet sind, wobei eine elektronische Schnittstelle (111), die an der kreiszylindrischen Aufnahmevorrichtung (10) vorliegt, und die elektronische Schnittstelle (11) des Bodens (3") in einer Betriebsanordnung, in der die Sensoreinheit (3) lösbar in der Ausnehmung (3') des Gehäuses (7) aufgenommen ist, miteinander in kommunikativer Verbindung stehen;
und wobei die Sensoreinheit (3) einen Deckel (20) mit einer Abdeckplatte (20') aufweist, die einen Testgaszugang (21) aufweist, durch den ein Fluidpfad (a) für ein Testgas zu dem Gassensor (60) bereitgestellt wird, und wobei
- die kreiszylindrische Ausnehmung (3') in dem Gehäusedeckel (4) des Gehäuses (7) erste Verriegelungsmittel und die Sensoreinheit (3) zweite Verriegelungsmittel aufweist, die mit den ersten Verriegelungsmitteln lösbar in Eingriff bringbar sind, und wobei das Gehäuse (7) und die kreiszylindrische Aufnahmevorrichtung (10) der Sensoreinheit (3) den Fluidpfad (b) für ein Messgas zu dem Gassensor (60) bereitstellen.

2. Gaswarngerät (1) nach Anspruch 1,
**dadurch gekennzeichnet, dass**
die Sensoreinheit (3) in der kreiszylindrischen Aufnahmevorrichtung (10) ein Messrohr (61) aufweist, das eine Messtrecke für ein Messgas in dem Fluidpfad (b) zu dem Gassensor (60) bereitstellt.

3. Gaswarngerät (1) nach Anspruch 1 oder 2,
**dadurch gekennzeichnet, dass**
die kreiszylindrische Aufnahmevorrichtung (10)
- ein Ringelement (65) mit einem Bodenabschnitt (165) aufweist, in dem zumindest eine Ausnehmung (165') vorliegt, durch die der Fluidpfad (b) für das Messgas verläuft, und die dazu ausgebildet ist, einen Durchtritt für die elektronische Schnittstelle (11) des Bodens (3") des Gehäusedeckels (4) zur Verbindung mit der elektronischen Schnittstelle (111) bereitzustellen,
- eine kreiszylindrische Grundplatte (70) aufweist, die außenumfänglich eine Mehrzahl Haltemittel, bevorzugt Haltebügel (71) aufweist, wobei in der kreiszylindrischen Grundplatte (70) eine Ausnehmung (70') vorliegt, die dazu ausgebildet ist, einen Durchtritt für die elektronische Schnittstelle (11) des Bodens (3") des Gehäusedeckels (4) zur Verbindung mit der elektronischen Schnittstelle (111) bereitzustellen, und wobei eine Mehrzahl Durchtrittsöffnungen (74), ausgebildet zum durchtreten Lassen eines Messgases, in der kreiszylindrischen Grundplatte (70) vorliegt, und wobei das Ringelement (65) auf der kreiszylindrischen Grundplatte (70) aufsitzt, bevorzugt auf einem verdickten Umfangsrand (70") der kreiszylindrischen Grundplatte (70).

4. Gaswarngerät (1) nach Anspruch 3,
**dadurch gekennzeichnet, dass**
das Ringelement (65) an seiner dem Bodenabschnitt (165) zugewandten Kante einen außenumfänglichen Flansch (65') aufweist, und
ein kreiszylindrischer Mantel (23) des Deckels (20), der die Abdeckplatte (20') trägt, sich gegen den Flansch (65`) abstützt, bevorzugt auf einem auf dem Flansch anliegenden Dichtmittel, besonders bevorzugt auf einem Dichtring (68).

5. Gaswarngerät (1) nach Anspruch 4,
**dadurch gekennzeichnet, dass**
ein Drehring (30) drehbar außenumfänglich um den Mantel (23) des Deckels (20) angeordnet ist, bevorzugt überkragt durch die Abdeckplatte (20'), und in der Betriebsanordnung um einem Rand (15') der Ausnehmung (3') in dem Gehäuse (7) anliegt, bevorzugt mittels eines außenumfänglichen Umgriffs (35) des Drehrings (30) drehbar aufliegt,
wobei der Drehring (30) zumindest zwei Typen Positionierelemente (33, 34), bevorzugt drei Typen Positionierelemente (33, 34, 36) aufweist, die in Zusammenwirkung mit Positionierelementen (15), die das Gehäuse (7) aufweist, eine Position des Drehrings (30) in Bezug zu der Ausnehmung (3') bestimmen.

6. Gaswarngerät (1) nach Anspruch 5,
**dadurch gekennzeichnet, dass**
die Positionierelemente aller Typen (33, 34, 36) erste Platten (34), zweite Platten (33) und/oder dritte Platten (36) sind, die sich von einer zu der kreiszylindrischen Grundplatte (70) weisenden Unterseite des Drehrings (30) in Richtung der kreiszylindrischen Grundplatte (70) erstrecken, wobei
- die ersten Platten (34) radial näher zu einem Mittelpunkt des Drehringes (30) an der Unterseite des Drehrings (30) angeordnet sind, bevorzugt an einem inneren Umfang des Drehrings (30), und durch Lücken (34') voneinander beabstandet sind, als die zweiten Platten (33), die ebenfalls durch Lücken (33') voneinander beabstandet an der Unterseite des Drehrings (30), bevorzugt an dessen äußerem Umfang, angeordnet sind, wobei bevorzugt die Lücken (34') zwischen den ersten Platten (34) und die Lücken (33') zwischen den zweiten Platten (33) nicht kongruent sind, und wobei besonders bevorzugt in Bezug auf eine axiale Erstreckung der Platten (33, 34) die zweiten Platten (33) kürzer sind als die ersten Platten (34).

7. Gaswarngerät (1) nach Anspruch 6,
**dadurch gekennzeichnet, dass**
die dritte Platten (36) sich von einer zu der kreiszylindrischen Grundplatte (70) weisenden Unterseite des Drehrings (30) in Richtung der kreiszylindrischen Grundplatte (70) erstrecken, wobei die dritten Platten (36) in Bezug auf die radiale Anordnung zu einem Mittelpunkt des Drehringes (30) zwischen den ersten Platten (34) und den zweiten Platten (33) angeordnet und durch Lücken (36') voneinander beabstandet an der Unterseite des Drehrings (30) angeordnet sind, wobei bevorzugt die Lücken (34') zwischen den ersten Platten (34) und die Lücken (33') zwischen den zweiten Platten (33) mit den Lücken (36') zwischen den dritten Platten (36) nicht kongruent sind und wobei besonders bevorzugt
in Bezug auf eine axiale Erstreckung der Platten (33, 34, 36) die dritten Platten (36) kürzer sind als die ersten Platten (34).

8. Gaswarngerät (1) nach zumindest einem der Ansprüche 5 bis 7,
**dadurch gekennzeichnet, dass**
die Positionierelemente (15) an dem Außenumfang des Randes (15') der Ausnehmung (3') verteilt und durch Lücken (15") voneinander beabstandet sind.

9. Gaswarngerät (1) nach zumindest einem der Ansprüche 1 bis 8,
**dadurch gekennzeichnet, dass**
Haltestege (12) auf dem Boden (3") an einer inneren Mantelfläche der Ausnehmung (3') des Gehäuses (7) verteilt vorliegen, die längsaxial in Bezug zu der Mantelfläche verlaufen, die mit den Haltemitteln der kreiszylindrischen Grundplatte (70), die bevorzugt Haltebügel (71) sind, zum lösbaren Halten der Sensoreinheit (3) in der Ausnehmung (3') in Eingriff stehen.

10. Gaswarngerät (1) nach zumindest einem der Ansprüche 1 bis 9,
**dadurch gekennzeichnet, dass**
auf dem Boden (3") des Gehäuses (7) in dem Gehäusedeckel (4) ein kreisförmiger Steg (18) vorliegt, der mit einem Abstand (A) von der inneren Mantelfläche der Ausnehmung (3') beabstandet ist,
und wobei die kreiszylindrische Grundplatte (70) der Sensoreinheit (3) an ihrer zu dem Boden (3") weisenden Seite umfangsnah eine Nut (75) aufweist, die fluidisch mit zumindest einem Teil der Durchtrittsöffnungen (74) der kreiszylindrischen Grundplatte (70) kommuniziert,
wobei die Nut (75) den kreisförmigen Steg (18) des Bodens (3") des Gehäuses (7) aufnimmt.

11. Gaswarngerät (1) nach zumindest einem der Ansprüche 5 bis 10,
**dadurch gekennzeichnet, dass**
die ersten Verriegelungsmittel, die die Ausnehmung (3') aufweist an einer inneren Mantelfläche vorliegende und radial nach innen weisende Elemente, bevorzugt Vorsprünge oder Nasen (14) sind,
und die zweiten Verriegelungsmittel, die die Sensoreinheit (3) aufweist, an zumindest zweien der Positionierelemente (34), die erste Platten (34) sind,
vorliegen, wobei die zweiten Verriegelungsmittel bevorzugt an einer Außenseite der ersten Platten (34) vorliegende Längsstege (14') sind, und wobei
bei einem Eingriff der ersten mit den zweiten Verriegelungsmitteln die Längsstege (14') die nach innen weisende Elemente, bevorzugt die Vorsprünge oder Nasen (14) untergreifen.

12. Gaswarngerät (1) nach zumindest einem der Ansprüche 5 bis 11,
**dadurch gekennzeichnet, dass**
der Drehring (30) in Bezug zu der Ausnehmung (3') in eine
- Offenposition bringbar ist, in der der Fluidpfad (b) von außerhalb des Gaswarngeräts (1) bis hin zu dem Gassensor (60) durchgängig ist, wobei in der Offenposition die Positionierelemente (15) und die Positionierelemente (33) miteinander fluchten,
- Geschlossenposition bringbar ist, in der der Fluidpfad (b) von außerhalb des Gaswarngeräts (1) bis hin zu dem Gassensor (60) nicht durchgängig ist, wobei die Positionierelemente (15) und die Positionierelemente (33) umfänglich in Bezug zu dem Rand (15') der Ausnehmung (3') zueinander versetzt positioniert sind,
- Austauschposition bringbar ist, in der die ersten mit den zweiten Verriegelungsmitteln nicht in Eingriff stehen.

13. Gaswarngerät (1) nach zumindest einem der Ansprüche 1 bis 12,
**dadurch gekennzeichnet, dass**
in der kreiszylindrischen Aufnahmevorrichtung (10) eine Batterie (17) angeordnet ist.

14. Gaswarngerät (1) nach zumindest einem der Ansprüche 1 bis 13,
**dadurch gekennzeichnet, dass**
eine Ausgabevorrichtung, bevorzugt eine optische und/oder eine akustische Ausgabevorrichtung, in der Sensoreinheit (3) und/oder an dem Gehäuse (7) vorliegt, die mit der Steuerungs- und Datenverarbeitungseinheit (50) kommunikativ verbunden ist und dazu ausgebildet ist, erfasste Messwerte des Gassensors (60) und/oder Positionsdaten, die von Positionssensoren erfasst werden, die an dem Gehäuse (7) vorliegen und dazu ausgebildet sind, eine jeweilige Position des Drehrings (30) zu erfassen, und die mit der Steuerungs- und Datenverarbeitungseinheit (50) kommunikativ verbunden sind, optisch und/oder akustisch auszugeben.

15. Gaswarngerät nach zumindest einem der Ansprüche 6-14, wobei der Fluidpfad (b) von außerhalb des Gaswarngeräts (1) entlang der sich überlappenden Lücken (33') und der Lücken (15") der Positionierelemente (15) und weiter der sich überlappenden Lücken (36') und der Lücken (34') und weiter entlang der Mantelfläche über den Steg (18) durch die Durchtrittsöffnungen (74) zu dem Gassensor (60) verläuft.

16. Gaswarnvorrichtung (100) mit zumindest einem Gaswarngerät (1) nach einem der Ansprüche 1 bis 15 und einer datenverarbeitenden Zentraleinheit (80),
**wobei**
das Gaswarngerät (1) mit der Zentraleinheit (80) kommunikativ verbindbar ist.

17. Verfahren zum Durchführen einer Wartungshandlung an einem Gaswarngerät (1) nach zumindest einem der Ansprüche 1 bis 15,
**umfassend die Schritte**
- die ersten Verriegelungsmittel, die die Ausnehmung (3') aufweist, und die zweiten Verriegelungsmittel, die die Sensoreinheit (3) aufweist, und die lösbar miteinander in Eingriff bringbar sind, voneinander Lösen,
- Entnehmen der Sensoreinheit (3) mit dem Gassensor (60) aus der Ausnehmung (3') des Gehäuses (7) und
- Einsetzen einer neuen Sensoreinheit (3) mit einem neuen Gassensor (60) in die Ausnehmung (3') des Gehäuses (7),
- die ersten Verriegelungsmittel, die die Ausnehmung (3') aufweist, und die zweiten Verriegelungsmittel, die die Sensoreinheit (3) aufweist, und die lösbar miteinander in Eingriff bringbar sind, miteinander in Eingriff bringen.

18. Verfahren zum Durchführen einer Gasmessung mit einem Gaswarngerät (1) nach zumindest einem der Ansprüche 5 bis 15,
wobei die Gasmessung ein Testen des Gassensors (60) mit einem Testgas oder ein Messen mit einem Messgas ist,
**umfassend die Schritte**
in einer ersten Phase
- den Drehring (30) in eine Geschlossenposition Bringen, in der der Fluidpfad (b) von außerhalb des Gaswarngeräts (1) bis hin zu dem Gassensor (60) nicht durchgängig ist, wobei die Positionierelemente (15) und die Positionierelemente (33) umfänglich in Bezug zu dem Rand (15') der Ausnehmung (3') zueinander versetzt positioniert sind,
- Zuführen eines Testgases durch den Testgaszugang (21) für das Testgas unter die Abdeckplatte (20'), durch den ein Fluidpfad (a) für das Testgas zu dem Gassensor (60) bereitgestellt wird, zu dem Gassensor (60),
- Erfassen des Testgases mit dem Gassensor (60) und Erzeugen eines Messsignals,
- Ausgeben eines mit dem Messsignal korrelierten Messwertes durch die Steuerungs- und Datenverarbeitungseinheit (50),
in einer zweiten Phase
- den Drehring (30) in eine Offenposition Bringen, in der der Fluidpfad (b) von außerhalb des Gaswarngeräts (1) bis hin zu dem Gassensor (60) durchgängig ist, wobei in der Offenposition die Positionierelemente (15) und die Positionierelemente (33) miteinander fluchten,
- strömen Lassen eines Messgases entlang dem Fluidpfad (b) zu dem Gassensor (60),
- Erfassen des Messgases mit dem Gassensor (60) und Erzeugen eines Messsignals,
- Ausgeben eines mit dem Messsignal korrelierten Messwertes durch die Steuerungs- und Datenverarbeitungseinheit (50),
wobei die erste und die zweite Phase auch in umgekehrter Reihenfolge oder in zeitlichem Abstand voneinander ausführbar sind.

## Claims

1. A gas warning unit (1), which has a sensor unit (3) releasably arranged therein, **wherein** the gas warning unit (1)
has a housing (7) comprising a housing cover (4), a housing body (5), and a housing bottom part (6), wherein a circular cylindrical recess (3') comprising a bottom (3"), which has an electronic interface (11), is present in the housing cover (4), and wherein
the sensor unit (3) has a circular cylindrical receiving device (10), in which a gas sensor (60) and at least one control and data processing unit (50), which is in communicative connection with the gas sensor (60), are arranged, wherein an electronic interface (11), which is present at the circular cylindrical receiving device (10), and the electronic interface (11) of the bottom (3") are in communicative connection with one another in an operating arrangement, in which the sensor unit (3) is releasably received in the recess (3') of the housing (7);
and wherein the sensor unit (3) has a cover (20) comprising a cover plate (20'), which has a test gas access (21), through which a fluid path (a) for a test gas to the gas sensor (60) is provided, and wherein
- the circular cylindrical recess (3') in the housing cover (4) of the housing (7) has first locking means and the sensor unit (3) has second locking means, which can be releasably engaged with the first locking means,
and wherein the housing (7) and the circular cylindrical receiving device (10) of the sensor unit (3) provide the fluid path (b) for a measuring gas to the gas sensor (60).

2. The gas warning unit (1) according to claim 1,
**characterized in that**
the sensor unit (3) in the circular cylindrical receiving device (10) has a measuring tube (61), which provides a measuring section for a measuring gas in the fluid path (b) to the gas sensor (60).

3. The gas warning unit (1) according to claim 1 or 2,
**characterized in that**
the circular cylindrical receiving device (10)
- has a ring element (65) comprising a bottom section (165), in which at least one recess (165') is present, through which the fluid path (b) for the measuring gas runs, and which is formed to provide a passage for the electronic interface (11) of the bottom (3") of the housing cover (4) for connection to the electronic interface (111),
- has a circular cylindrical base plate (70), which has a plurality of retaining means, preferably retaining brackets (71) on the outer circumference, wherein a recess (70') is present in the circular cylindrical base plate (70), which is formed to provide a passage for the electronic interface (11) of the bottom (3") of the housing cover (4) for connection to the electronic interface (111),
and wherein a plurality of passage openings (74), formed for allowing a measuring gas to pass, is present in the circular cylindrical base plate (70), and
wherein the ring element (65) sits on the circular cylindrical base plate (70), preferably on a thickened circumferential edge (70") of the circular cylindrical base plate (70).

4. The gas warning unit (1) according to claim 3,
**characterized in that**
the ring element (65) has an outer circumferential flange (65') on its edge facing the bottom section (165), and
a circular cylindrical jacket (23) of the cover (20), which carries the cover plate (20'), is supported against the flange (65'), preferably on a sealing means abutting on the flange, particularly preferably on a sealing ring (68).

5. The gas warning unit (1) according to claim 4,
**characterized in that**
a rotary ring (30) is rotatably arranged around the jacket (23) of the cover (20) on the outer circumference, preferably protruded by the cover plate (20'), and abuts around an edge (15') of the recess (3') in the housing (7) in the operating arrangement, preferably rotatably bears on by means of an outer circumferential wrap-around (35) of the rotary ring (30),
wherein the rotary ring (30) has at least two types of positioning elements (33, 34), preferably three types of positioning elements (33, 34, 36), which, in cooperation with positioning elements (15), which the housing (7) has, determine a position of the rotary ring (30) with respect to the recess (3').

6. The gas warning unit (1) according to claim 5,
**characterized in that**
the positioning elements of all types (33, 34, 36) are first plates (34), second plates (33) and/or third plates (36), which extend from an underside of the rotary ring (30) facing the circular cylindrical base plate (70) in the direction of the circular cylindrical base plate (70), wherein
- the first plates (34) are arranged radially closer to a center point of the rotary ring (30) on the underside of the rotary ring (30), preferably on an inner circumference of the rotary ring (30), and are spaced apart from one another by means of gaps (34'), than the second plates (33), which are likewise arranged spaced apart from one another by means of gaps (33') on the underside of the rotary ring (30), preferably on the outer circumference thereof, wherein the gaps (34') between the first plates (34) and the gaps (33') between the second plates (33) are preferably not congruent, and wherein the second plates (33) are shorter than the first plates (34) particularly preferably with respect to an axial extension of the plates (33, 34).

7. The gas warning unit (1) according to claim 6,
**characterized in that**
the third plates (36) extend from an underside of the rotary ring (30) facing the circular cylindrical base plate (70) in the direction of the circular cylindrical base plate (70),
wherein the third plates (36) are arranged between the first plates (34) and the second plates (33) with respect to the radial arrangement to a center point of the rotary ring (30) and are arranged spaced apart from one another by means of gaps (36') on the underside of the rotary ring (30), wherein the gaps (34') between the first plates (34) and the gaps (33') between the second plates (33) are preferably not congruent with the gaps (36') between the third plates (36), and wherein
the third plates (36) are particularly preferably shorter than the first plates (34) with respect to an axial extension of the plates (33, 34, 36).

8. The gas warning unit (1) according to at least any one of claims 5 to 7,
**characterized in that**
the positioning elements (15) are distributed on the outer circumference of the edge (15') of the recess (3') and are spaced apart from one another by means of gaps (15").

9. The gas warning unit (1) according to at least any one of claims 1 to 8,
**characterized in that**
retaining webs (12), which run longitudinally axially with respect to the jacket surface and which engage with the retaining means of the circular cylindrical base plate (70), which are preferably retaining brackets (71), are present on the bottom (3") so as to be distributed on an inner jacket surface of the recess (3') of the housing (7), for releasably retaining the sensor unit (3) in the recess (3').

10. The gas warning unit (1) according to at least any one of claims 1 to 9,
**characterized in that**
a circular web (18), which is spaced apart from the inner jacket surface of the recess (3') at a distance (A), is present on the bottom (3") of the housing (7) in the housing cover (4),
and wherein the circular cylindrical base plate (70) of the sensor unit (3) has, on its side facing the bottom (3") close to the circumference, a groove (75), which fluidically communicates with at least a portion of the passage openings (74) of the circular cylindrical base plate (70),
wherein the groove (75) receives the circular web (18) of the bottom (3") of the housing (7).

11. The gas warning unit (1) according to at least any one of claims 5 to 10,
**characterized in that**
the first locking means, which the recess (3') has, are elements, preferably protrusions or lugs (14), which are present on an inner jacket surface and which face radially inwards,
and the second locking means, which the sensor unit (3) has, are present on at least two of the positioning elements (34), which are first plates (34), wherein the second locking means are preferably longitudinal webs (14'), which are present on an outer side of the first plates (34), and wherein
in response to an engagement of the first with the second locking means, the longitudinal webs (14') engage below the elements facing inwards, preferably the protrusions or lugs (14).

12. The gas warning unit (1) according to at least any one of claims 5 to 11,
**characterized in that**
with respect to the recess (3'), the rotary ring (30) can be
- brought into an open position, in which the fluid path (b) is continuous from outside the gas warning unit (1) all the way to the gas sensor (60), wherein the positioning elements (15) and the positioning elements (33) are aligned with one another in the open position,
- brought into a closed position, in which the fluid path (b) is not continuous from outside the gas warning unit (1) all the way to the gas sensor (60), wherein the positioning elements (15) and the positioning elements (33) are positioned to one another circumferentially with respect to the edge (15') of the recess (3'),
- brought into a replacement position, in which the first locking means do not engage with the second locking means.

13. The gas warning unit (1) according to at least any one of claims 1 to 12,
**characterized in that**
a battery (17) is arranged in the circular cylindrical receiving device (10).

14. The gas warning unit (1) according to at least any one of claims 1 to 13,
**characterized in that**
an output device, preferably an optical and/or an acoustic output device, is present in the sensor unit (3) and/or on the housing (7), which is communicatively connected to the control and data processing unit (50) and which is formed to optically and/or acoustically output detected measured values of the gas sensor (60) and/or position data, which is detected by position sensors, which are present on the housing (7) and which are formed to detect a respective position of the rotary ring (30), and which are communicatively connected to the control and data processing unit (50).

15. The gas warning unit according to at least any one of claims 6-14, wherein the fluid path (b) runs from outside the gas warning unit (1) along the overlapping gaps (33') and the gaps (15") of the positioning elements (15) and further of the overlapping gaps (36') and the gaps (34') and further along the jacket surface over the web (18) through the passage openings (74) to the gas sensor (60).

16. A gas warning device (100) comprising at least one gas warning unit (1) according to any one of claims 1 to 15 and a data processing central unit (80),
**wherein**
the gas warning unit (1) can be communicatively connected to the central unit (80).

17. A method for carrying out a maintenance action on a gas warning unit (1) according to at least any one of claims 1 to 15,
**comprising the steps of**
- releasing the first locking means, which the recess (3') has, and the second locking means, which the sensor unit (3) has, and which can be releasably engaged with one another, from one another,
- removing the sensor unit (3) comprising the gas sensor (60) from the recess (3') of the housing (7), and
- inserting a new sensor unit (3) comprising a new gas sensor (60) into the recess (3') of the housing (7),
- engaging the first locking means, which the recess (3') has, and the second locking means, which the sensor unit (3) has, and the second locking means, which the sensor unit (3) has, and which can be releasably engaged with one another, with one another.

18. A method for carrying out a gas measurement by means of a gas warning unit (1) according to at least any one of claims 5 to 15,
wherein the gas measurement is a testing of the gas sensor (60) by means of a test gas or a measurement by means of a measuring gas,
**comprising the steps of**
in a first phase,
- bringing the rotary ring (30) into a closed position, in which the fluid path (b) is not continuous from outside the gas warning unit (1) all the way to the gas sensor (60), wherein the positioning elements (15) and the positioning elements (33) are positioned offset from one another circumferentially with respect to the edge (15') of the recess (3'),
- supplying a test gas through the test gas access (21), through which a fluid path (a) for the test gas to the gas sensor (60) is provided, for the test gas under the cover plate (20'), to the test gas sensor (60),
- detecting the test gas by means of the gas sensor (60) and generating a measuring signal,
- outputting a measured value, which correlates with the measuring signal, by means of the control and data processing unit (50),
in a second phase,
- bringing the rotary ring (30) into an open position, in which the fluid path (b) is continuous from outside the gas warning unit (1) all the way to the gas sensor (60), wherein the positioning elements (15) and the positioning elements (33) are aligned with one another in the open position,
- allowing a measuring gas to flow along the fluid path (b) to the gas sensor (60),
- detecting the measuring gas by means of the gas sensor (60) and generating a measuring signal,
- outputting a measured value, which correlates with the measuring signal, by means of the control and data processing unit (50),
wherein the first and the second phase can also be carried out in reverse order or at a time interval from one another.

## Revendications

1. Détecteur de gaz (1) comprenant une unité capteur (3) disposée de manière amovible dans celui-ci,
le détecteur de gaz (1) comprenant
un boîtier (7) avec un couvercle de boîtier (4), un corps de boîtier (5) et une partie inférieure de boîtier (6), un évidement cylindrique circulaire (3') avec un fond (3"), lequel présente une interface électronique (11), étant présent dans le couvercle de boîtier (4), et
l'unité capteur (3) disposant d'un dispositif de réception cylindrique circulaire (10), dans lequel sont disposés un capteur de gaz (60) et au moins une unité de commande et de traitement de données (50) qui est en liaison de communication avec le capteur de gaz (60), une interface électronique (111) qui est présente sur le dispositif de réception cylindrique circulaire (10), et l'interface électronique (11) du fond (3") étant en liaison de communication l'une avec l'autre dans une disposition de fonctionnement dans laquelle l'unité capteur (3) est reçue de manière amovible dans l'évidement (3') du boîtier (7) ;
et l'unité capteur (3) comprenant un couvercle (20) avec une plaque de protection (20') qui comprend un accès au gaz d'essai (21) par lequel un trajet de fluide (a) pour un gaz d'essai est fourni au capteur de gaz (60), et
- l'évidement cylindrique circulaire (3') dans le couvercle du boîtier (4) du boîtier (7) comprenant des premiers moyens de verrouillage et l'unité capteur (3) comprenant des deuxièmes moyens de verrouillage qui peuvent être mis en prise de manière amovible avec les premiers moyens de verrouillage, et le boîtier (7) et le dispositif de réception cylindrique circulaire (10) de l'unité capteur (3) fournissant le trajet de fluide (b) pour un gaz de mesure vers le capteur de gaz (60).

2. Détecteur de gaz (1) selon la revendication 1,
**caractérisé en ce que**
l'unité capteur (3) comprend, dans le dispositif de réception cylindrique circulaire (10), un tube de mesure (61) qui fournit une voie de mesure pour un gaz de mesure dans le trajet de fluide (b) vers le capteur de gaz (60).

3. Détecteur de gaz (1) selon la revendication 1 ou 2,
**caractérisé en ce que**
le dispositif de réception cylindrique circulaire (10) comporte
- un élément annulaire (65) avec une section de fond (165) dans laquelle se trouve au moins un évidement (165') par lequel passe le trajet de fluide (b) pour le gaz de mesure, et qui est conçu pour fournir un passage pour l'interface électronique (11) du fond (3") du couvercle de boîtier (4) pour la connexion avec l'interface électronique (111),
- une plaque de base cylindrique circulaire (70), qui présente à l'extérieur une pluralité de moyens de retenue, préférablement des supports de retenue (71), un évidement (70') étant présent dans la plaque de base cylindrique circulaire (70), lequel est conçu pour fournir un passage pour l'interface électronique (11) du fond (3") du couvercle de boîtier (4) pour la liaison avec l'interface électronique (111), et une pluralité d'ouvertures de passage (74), conçues pour laisser passer un gaz de mesure, étant présentes dans la plaque de base cylindrique circulaire (70), et l'élément annulaire (65) reposant sur la plaque de base cylindrique circulaire (70), préférablement sur un bord périphérique épaissi (70") de la plaque de base cylindrique circulaire (70).

4. Détecteur de gaz (1) selon la revendication 3,
**caractérisé en ce que**
l'élément annulaire (65) comporte, sur son bord tourné vers la section de fond (165), un flasque (65') extérieur, et une enveloppe cylindrique circulaire (23) du couvercle (20) qui supporte la plaque de protection (20'), s'appuie contre le flasque (65'), préférablement sur un moyen d'étanchéité reposant sur le flasque, plus préférablement sur une joint d'étanchéité (68).

5. Détecteur de gaz (1) selon la revendication 4,
**caractérisé en ce que**
un anneau rotatif (30) est disposé de manière à pouvoir tourner à l'extérieur autour de l'enveloppe (23) du couvercle (20), préférablement surplombé par la plaque de protection (20'), et s'appuie dans la disposition de fonctionnement autour d'un bord (15') de l'évidement (3') dans le boîtier (7), préférablement repose de manière à pouvoir tourner au moyen d'un cadre (35) extérieur de l'anneau rotatif (30), l'anneau rotatif (30) comportant au moins deux types d'éléments de positionnement (33, 34), préférablement trois types d'éléments de positionnement (33, 34, 36), qui, en interaction avec les éléments de positionnement (15) que comporte le boîtier (7), déterminent une position de l'anneau rotatif (30) par rapport à l'évidement (3').

6. Détecteur de gaz (1) selon la revendication 5,
**caractérisé en ce que**
les éléments de positionnement de tous types (33, 34, 36) sont les premières plaques (34), les deuxièmes plaques (33) et/ou les troisièmes plaques (36) qui s'étendent depuis une face inférieure de l'anneau rotatif (30), orientée vers la plaque de base cylindrique circulaire (70), en direction de la plaque de base cylindrique circulaire (70),
- les premières plaques (34) étant disposées radialement plus près d'un centre de l'anneau rotatif (30) sur la face inférieure de l'anneau rotatif (30), préférablement sur un pourtour intérieur de l'anneau rotatif (30), et étant espacées les unes des autres par des espaces (34'), que les deuxièmes plaques (33) qui sont également espacées les unes des autres par des espaces (33') sur la face inférieure de l'anneau rotatif (30), préférablement sur son pourtour extérieur, les espaces (34') entre les premières plaques (34) et les espaces (33') entre les deuxièmes plaques (33) n'étant préférablement pas congruents, et plus préférablement par rapport à une extension axiale des plaques (33, 34), les deuxièmes plaques (33) étant plus courtes que les premières plaques (34).

7. Détecteur de gaz (1) selon la revendication 6,
**caractérisé en ce que**
les troisièmes plaques (36) s'étendent, depuis une face inférieure de l'anneau rotatif (30), orientée vers la plaque de base cylindrique circulaire (70), en direction de la plaque de base cylindrique circulaire (70), les troisièmes plaques (36) étant disposées entre les premières plaques (34) et les deuxièmes plaques (33) en ce qui concerne la disposition radiale par rapport à un centre de l'anneau rotatif (30) et étant disposées sur la face inférieure de l'anneau rotatif (30) en étant espacées les unes des autres par des espaces (36'), les espaces (34') entre les premières plaques (34) et les espaces (33') entre les deuxièmes plaques (33) n'étant préférablement pas congruents avec les espaces (36') entre les troisièmes plaques (36), et plus préférablement
par rapport à une extension axiale des plaques (33, 34, 36), les troisièmes plaques (36) étant plus courtes que les premières plaques (34).

8. Détecteur de gaz (1) selon au moins l'une des revendications 5 à 7,
**caractérisé en ce que**
les éléments de positionnement (15) sont répartis sur le pourtour extérieur du bord (15') de l'évidement (3') et espacés les uns des autres par des espaces (15").

9. Détecteur de gaz (1) selon au moins l'une des revendications 1 à 8,
**caractérisé en ce que**
entretoises (12) sont réparties sur le fond (3") sur une surface d'enveloppe intérieure de l'évidement (3') du boîtier (7), lesquelles s'étendent dans l'axe longitudinal par rapport à la surface d'enveloppe, sont en prise avec les moyens de retenue de la plaque de base cylindrique circulaire (70), qui sont préférablement des supports de retenue (71), pour le maintien amovible l'unité capteur (3) dans l'évidement (3').

10. Détecteur de gaz (1) selon au moins l'une des revendications 1 à 9,
**caractérisé en ce que**
sur le fond (3") du boîtier (7) dans le couvercle de boîtier (4), il y a une entretoise circulaire (18) qui est espacée d'une distance (A) de la surface d'enveloppe intérieure de l'évidement (3'),
et la plaque de base cylindrique circulaire (70) de l'unité capteur (3) présentant, sur son côté orienté vers le fond (3"), à proximité du pourtour, une rainure (75) qui communique par voie fluidique avec au moins une partie des ouvertures de passage (74) de la plaque de base cylindrique circulaire (70),
la rainure (75) recevant l'entretoise circulaire (18) du fond (3") du boîtier (7).

11. Détecteur de gaz (1) selon au moins l'une des revendications 5 à 10,
**caractérisé en ce que**
les premiers moyens de verrouillage que comporte l'évidement (3'), sont des éléments, préférablement des saillies ou des ergots (14), présents sur une surface d'enveloppe intérieure et orientés radialement vers l'intérieur,
et les deuxièmes moyens de verrouillage que comporte l'unité capteur (3), sur au moins deux des éléments de positionnement (34), sont des premières plaques (34), les deuxièmes moyens de verrouillage étant préférablement des entretoises longitudinales (14') présentes sur un côté extérieur des premières plaques (34), et lors de l'engagement des premiers moyens de verrouillage avec les deuxièmes moyens de verrouillage, les entretoises longitudinales (14') s'engageant sous les éléments orientés vers l'intérieur, préférablement les saillies ou les ergots (14).

12. Détecteur de gaz (1) selon au moins l'une des revendications 5 à 11,
**caractérisé en ce que**
l'anneau rotatif (30) peut être placé, par rapport à l'évidement (3'), dans une
- position ouverte dans laquelle le trajet de fluide (b) est continu depuis l'extérieur du détecteur de gaz (1) jusqu'au capteur de gaz (60), les éléments de positionnement (15) et les éléments de positionnement (33) étant alignés dans la position ouverte,
- position fermée dans laquelle le trajet de fluide (b) n'est pas continu depuis l'extérieur du détecteur de gaz (1) jusqu'au capteur de gaz (60), les éléments de positionnement (15) et les éléments de positionnement (33) étant décalés les uns des autres sur le pourtour par rapport au bord (15') de l'évidement (3'),
- position de remplacement dans laquelle les premiers et les deuxièmes moyens de verrouillage ne sont pas en prise.

13. Détecteur de gaz (1) selon au moins l'une des revendications 1 à 12,
**caractérisé en ce que**
une batterie (17) est disposée dans le dispositif de réception cylindrique circulaire (10).

14. Détecteur de gaz (1) selon au moins l'une des revendications 1 à 13,
**caractérisé en ce que**
un dispositif d'émission, préférablement un dispositif d'émission optique et/ou acoustique, est présent dans l'unité capteur (3) et/ou sur le boîtier (7), lequel est en liaison de communication avec l'unité de commande et de traitement des données (50) et est conçu pour émettre de manière optique et/ou acoustique des valeurs de mesure enregistrées du capteur de gaz (60) et/ou des données de position qui sont enregistrées par des capteurs de position qui sont présents sur le boîtier (7), conçus pour enregistrer une position respective de l'anneau rotatif (30) et en liaison de communication avec l'unité de commande et de traitement des données (50).

15. Détecteur de gaz (1) selon au moins l'une des revendications 6 à 14, le trajet de fluide (b) s'étendant de l'extérieur du détecteur de gaz (1) le long des espaces (33') qui se chevauchent et des espaces (15") des éléments de positionnement (15) et plus loin des espaces (36') qui se chevauchent et des espaces (34') et plus loin le long de la surface d'enveloppe sur l'entretoise (18) par les ouvertures de passage (74) vers le capteur de gaz (60).

16. Dispositif de détection de gaz (100) comprenant au moins un détecteur de gaz (1) selon l'une des revendications 1 à 15 et une unité centrale de traitement de données (80),
le détecteur de gaz (1) pouvant être mis en liaison de communication avec l'unité centrale (80).

17. Procédé de réalisation d'une opération de maintenance sur un détecteur de gaz (1) selon au moins l'une des revendications 1 à 15,
**comprenant les étapes**
- séparation des premiers moyens de verrouillage que comporte l'évidement (3') et des deuxièmes moyens de verrouillage que comporte l'unité capteur (3), et qui peuvent être mis en prise de manière amovible,
- retrait de l'unité capteur (3) avec le capteur de gaz (60) de l'évidement (3') du boîtier (7) et
- insertion d'une nouvelle unité capteur (3) avec un nouveau capteur de gaz (60) dans l'évidement (3') du boîtier (7),
- mise en prise des premiers moyens de verrouillage que comporte l'évidement (3') et des deuxièmes moyens de verrouillage que comporte l'unité capteur (3), et qui peuvent être mis en prise de manière amovible.

18. Procédé de réalisation d'une une mesure de gaz avec un détecteur de gaz (1) selon au moins l'une des revendications 5 à 15,
la mesure du gaz étant un test du capteur de gaz (60) avec un gaz d'essai ou une mesure avec un gaz de mesure,
**comprenant les étapes**
dans une première phase
- mise de l'anneau rotatif (30) dans une position fermée dans laquelle le trajet de fluide (b) n'est pas continu de l'extérieur du détecteur de gaz (1) jusqu'au capteur de gaz (60), les éléments de positionnement (15) et les éléments de positionnement (33) étant décalés les uns des autres sur le pourtour par rapport au bord (15') de l'évidement (3'),
- fourniture d'un gaz d'essai vers le capteur de gaz (60) par l'accès au gaz d'essai (21) pour le gaz d'essai sous la plaque de protection (20'), par lequel un trajet de fluide (a) est fourni vers le capteur de gaz (60) pour le gaz d'essai,
- détection du gaz d'essai avec le capteur de gaz (60) et génération d'un signal de mesure,
- émission d'une valeur de mesure corrélée au signal de mesure par l'unité de commande et de traitement des données (50),
dans une deuxième phase
- mise de l'anneau rotatif (30) dans une position ouverte dans laquelle le trajet de fluide (b) est continu de l'extérieur du détecteur de gaz (1) jusqu'au capteur de gaz (60), les éléments de positionnement (15) et les éléments de positionnement (33) étant, en position ouverte, alignés les uns avec les autres,
- écoulement d'un gaz de mesure le long du trajet de fluide (b) vers le capteur de gaz (60),
- détection du gaz de mesure avec le capteur de gaz (60) et génération d'un signal de mesure,
- émission d'une valeur de mesure corrélée au signal de mesure par l'unité de commande et de traitement des données (50),
les première et la deuxième phases pouvant également être exécutées dans l'ordre inverse ou à un certain intervalle de temps.
